# EUROPÄISCHE PATENTSCHRIFT

(11) **EP 0 590 530 B1**
(45) Veröffentlichungstag und Bekanntmachung des Hinweises auf die Patenterteilung: **26.04.2000**
(21) Anmeldenummer: 93115418.1
(22) Anmeldetag: 24.09.1993
(51) Int. Cl.: C12N 15/62, C07K 14/00, C12N 9/00, C12N 15/81, A01K 67/027, A61K 38/00, G01N 33/68, C12N 1/21, C12N 5/10

(54) **Fusionsproteine zur Prodrug-Aktivierung**
Fusion proteins for prodrug-activation
Protéines de fusion pour l'activation de pro-médicaments

(30) Priorität: 02.10.1992 DE 4233152
(43) Veröffentlichungstag der Anmeldung: 06.04.1994
(73) Patentinhaber: HOECHST AKTIENGESELLSCHAFT, 65926 Frankfurt am Main (DE)
(72) Erfinder: Gehrmann, Mathias, D-35457 Lollar (DE); Seemann, Gerhard, D-35041 Marburg (DE); Bosslet, Klaus, D-35037 Marburg (DE); Czech, Jörg, D-35041 Marburg (DE)
(74) Vertreter: Fischer, Hans-Jürgen, Dr.

(56) Entgegenhaltungen:
- EP-A- 0 501 215
- WO-A-89/10140
- BRITISH JOURNAL OF CANCER, Bd. 65, Nr. 2, Februar 1992, Seiten 234-238, XP000612143 K.BOSSLET ET AL.: "Molecular and functional characterisation of a fusion protein suited for tumour specific prodrug activation"
- PROCEEDINGS OF THE NATIONAL ACADEMY OF SCIENCES OF USA, Bd. 89, Nr. 7, 1.April 1992, WASHINGTON US, Seiten 3075-3079, XP000264181 U.BRINKMANN ET AL.: "Independent domain folding of Pseudomonas exotoxin and single-chain immunotoxins: Influence of interdomain connections"

## Beschreibung

Die Erfindung betrifft Verbindungen, die eine Antigenbinderegion enthalten, welche an mindestens ein Enzym gebunden ist, das eine nicht oder wenig zytotoxische Verbindung (Prodrug) in eine zytotoxische Verbindung (Drug) metabolisieren kann, wobei die Antigenbinderegion aus einer einzigen Polypeptidkette besteht. Vorteilhafterweise befinden sich an der Polypeptidkette kovalent gebundene Kohlenhydrate.

Die Kombination von Prodrug und Antikörper-Enzym-Konjugaten zur Anwendung als therapeutisches Mittel ist in der Fachliteratur bereits beschrieben. Hierbei werden gegen ein bestimmtes Gewebe gerichtete Antikörper, an die ein Prodrug-spaltendes Enzym gebunden ist, einem Organismus injiziert, und anschließend wird eine enzym-aktivierbare Prodrug-Verbindung verabreicht. Unter der Einwirkung des am Zielgewebe gebundenen Antikörper-Enzym-Konjugates soll die Prodrug-Verbindung in eine Verbindung umgewandelt werden, die eine zytotoxische Wirkung gegen das gebundene Gewebe ausübt. Allerdings hat sich bei Arbeiten mit Antikörper-Enzym-Konjugaten gezeigt, daß diese chemischen Konjugate eine ungünstige Pharmakokinetik besitzen, so daß eine ortsspezifische tumorselektive Spaltung der Prodrug nur unzureichend erfolgt. Manche Autoren haben versucht, diesen offensichtlichen Mangel durch zusätzliche Injektion eines anti-Enzymantikörpers, der eine schnelle Eliminierung des Antikörperenzymkonjugates aus dem Plasma bewirken soll, zu beheben (Sharma et al., Brit. J. Cancer, 61, 659, 1990). Ein weiteres Problem von Antikörperenzymkonjugaten ist die begrenzte Möglichkeit, große Mengen reproduzierbar und homogen herzustellen.

Aufgabe der vorliegenden Erfindung war nun, Fusionsproteine zu finden, die in großtechnischem Maßstab hergestellt werden können und aufgrund ihrer pharmakokinetischen und pharmakodynamischen Eigenschaften für therapeutische Anwendungen geeignet sind.

Es wurde dabei gefunden, daß Verbindungen, die eine Antigenbinderegion enthalten, welche aus einer einzigen Polypeptidkette besteht, für die Herstellung und Verwendung von Fusionsproteinen, welche vorteilhaft mit Kohlenhydraten besetzt sind, bei der Prodrug-Aktivierung unervartete Vorteile besitzen.

Gegenstand der Erfindung sind daher Verbindungen, die eine Antigenbinderegion enthalten, welche an mindestens ein Enzym gebunden ist, wobei die Antigenbinderegion aus einer einzigen Polypeptidkette besteht und das Fusionsprotein vorteilhaft mit Kohlenhydraten besetzt ist.

Unter Antigenbinderegion versteht man im Sinne der Erfindung eine Region, die mindestens zwei variable Domänen eines Antikörpers enthält, vorzugsweise eine variable Domäne einer schweren Antikörperkette und eine variable Domäne einer leichten Antikörperkette (sFv-Fragment). Die Antigenbinderegion kann jedoch auch bi- oder multivalent aufgebaut sein, d.h. zwei oder mehr Binderegionen besitzen, wie beispielweise in der EP-A-0 404 097 offenbart. Besonders bevorzugt ist jedoch ein humanes oder humanisiertes sFv-Fragment, insbesondere ein humanisiertes sFv-Fragment.

Vorzugsweise bindet die Antigenbinderegion an ein tumorassoziiertes Antigen (TAA), wobei insbesondere folgende TAAs bevorzugt sind:
neural cell adhesion molecule (N-CAM),
polymorphic epithelial mucin (PEM),
epidermal growth factor receptor (EGF-R),
Thomsen Friedenreich antigen β (TFβ),
gastrointestinal tract carcinoma antigen (GICA),
ganglioside GD₃ (GD₃),
ganglioside GD₂ (GD₂),
Sialyl-Le^{a}, Sialyl-Le^{x},
TAG72,
das durch MAk L6 definierte Glykoprotein mit 24-25 kDa,
CA 125 und vor allem das
carcinoembryonic antigen (CEA).

Als Enzyme sind diejenigen Enzyme bevorzugt, die eine nicht oder wenig zytotoxische Verbindung in eine zytotoxische Verbindung metabolisieren können. Beispiele sind die β-Lactamase, Pyroglutamat-Aminopeptidase, Galactosidase oder D-Aminopeptidase wie z.B. in der EP-A2-0 382 411 oder EP-A2-0 392 745 beschrieben, eine Oxidase wie z.B. Ethanoloxidase, Galactoseoxidase, D-Aminosäureoxidase oder α-Glyceryl-Phosphatoxidase, wie z.B. in der WO 91/00108 beschrieben, eine Peroxidase gemäß z.B. EP-A2-0 361 908, eine Phosphatase, wie z.B. in EP-A1-0 302 473 beschrieben, eine Hydroxynitrillyase oder Glucosidase gemäß z.B. WO 91/11201, eine Carboxypeptidase, wie z.B. die Carboxypeptidase G2 (WO 88/07378), eine Amidase, wie z.B. die Penicillin-5-amidase (Kerr, D. E. et al. Cancer Immunol. Immunther. 1990, 31), eine Protease, Esterase oder Glycosidase, wie die bereits erwähnte Galactosidase, Glucosidase oder eine Glucuronidase, wie z.B. in der WO 91/08770 beschrieben. Bevorzugt ist eine β-Glucuronidase, vorzugsweise aus *Kobayasia nipponica oder Secale cereale* und besonders bevorzugt aus E. coli oder eine humane β-Glucuronidase. Die Substrate der einzelnen Enzyme sind in den genannten Schutzrechten mit angegeben und sollen auch zum Offenbarungsgehalt der vorliegenden Anmeldung gehören. Bevorzugte Substrate der β-Glucuronidase sind N-(D-Glycopyranosyl)-benzyloxycarbonyl-anthracycline und insbesondere das N-(4-Hydroxy-3-nitro-benzyloxycarbonyl)-doxorubicin bzw. daunorubicin-β-D-glucuronid (J. C. Florent et al. (1992) Int. Carbohydr. Symp. Paris, A262, 297 oder S. Andrianomenjanahary et al. (1992) Int. Carbohydr. Symp. Paris, A 264, 299).

Ein weiterer Gegenstand der Erfindung sind Nukleinsäuren, die für die erfindungsgemäßen Verbindungen kodieren. Insbesondere bevorzugt ist eine Nukleinsäure sowie deren Varianten und Mutanten, die für ein humanisiertes sFv-Fragment gegen CEA (Carcinoembryonales Antigen) verbunden mit einer humanen β-Glucuronidase kodiert (sFv-huβ-Gluc), vorzugsweise mit der in Tabelle 1 angegebenen Sequenz.

Die Herstellung der erfindungsgemäßen Verbindungen erfolgt im allgemeinen gentechnisch, nach dem Fachmann allgemein bekannten Verfahren, wobei die Antigenbinderegion mit einem oder mehreren Enzymen entweder direkt oder über einen Linker, vorzugsweise einem Peptidlinker, verbunden sein kann. Als Peptidlinker kann beispielsweise eine "Hinge-Region" eines Antikörpers oder eine "hinge"-ähnliche Aminosäuresequenz verwendet werden. Das Enzym ist dabei vorzugsweise mit dem N-Terminus an die Antigenbinderegion direkt oder über einen Peptidlinker verbunden. Das oder die Enzyme können jedoch auch chemisch, wie z.B. in der WO 91/00108 offenbart, mit der Antigenbinderegion verbunden werden.

Die für die Aminosäuresequenz der erfindungsgemäßen Verbindungen kodierende Nukleinsäure, wird im allgemeinen in einem Expressionsvektor kloniert, in prokaryontischen oder eukaryontischen Wirtszellen, wie z.B. BHK- CHO-, COS-, HeLa-, Insekten-, Tabakpflanzen-, Hefe- oder *E.coli*-Zellen eingebracht und exprimiert. Die so hergestellte Verbindung kann anschließend isoliert und als Diagnostikum oder Therapeutikum verwendet werden. Ein weiteres allgemein bekanntes Verfahren zur Herstellung der erfindungsgemäßen Verbindung ist die Expression der dafür kodierenden Nukleinsäuren in transgenen Säugern mit Ausnahme von Mensch, vorzugsweise in einer transgenen Ziege.

Mit den erfindungsgemäßen Nukleinsäuren transfizierte BHK-Zellen exprimierten ein Fusionsprotein (sFv-huβ-Gluc), welches sowohl spezifisch für CEA war als auch volle β-Glucuronidase Aktivität besitzt (siehe Bsp. 5).

Dieses Fusionsprotein wurde über Anti-idiotyp Affinitätschromatographie entsprechend der in EP O 501 215 A2 (Beispiel M) beschriebenen Methode gereinigt. Das so gereinigte Fusionsprotein besitzt unter reduzierenden Bedingungen in der SDS-PAGE ein Molekulargewicht von 100 kDa, unter nichtreduzierenden Bedingungen treten Moleküle von 100 bzw. 200 kDa auf.

Gelchromatographie unter nativen Bedingungen (TSK-3000 Gelchromatographie) zeigte einen Protein-Peak (Bsp. 6, Abb. I), der mit dem Aktivitätspeak im Spezifitätsenzymaktivitätstest (EP O 501 215 A2) korreliert. Die Position des Peaks im Vergleich zu Standard-Molekulargwichtsmarkern deutet auf ein Molekulargewicht von ≈ 200 kDa hin. Dieser Befund, verbunden mit den Daten aus der SDS-PAGE, suggeriert, daß das funktionelle, enzymatisch aktive sFv-huβ-Gluc Fusionsprotein als "bivalentes Molekül", d.h. mit 2 Binderegionen und 2 Enzymmolekülen, vorliegt. Hier nicht beschriebene Versuche deuten darauf hin, daß das Fusionsprotein unter bestimmten Kultivierungsbedingungen auch als Tetramer, mit 4 Binderegionen und 4 Enzymmolekülen, vorliegen kann. Nachdem das sFv-huβ-Gluc Fusionsprotein gereinigt und in vitro funktionell charakterisiert war, wurde die Pharmakokinetik und die Tumorlokalisation des Fusionsproteins in nackten Mäusen bestimmt, die mit menschlichen Magenkarzinomen bestückt waren. Die Mengen an funktionell aktivem Fusionsprotein wurden in den Organen sowie dem Tumor zu unterschiedlichen Zeitpunkten nach adäquater Aufarbeitung der Organe (Beispiel 7) sowie immunologischer Bestimmung (Triple-Determinanten-Test, Beispiel 8) bestimmt. Die Ergebnisse eines repräsentativen Versuches sind in Tabelle 4 zusammengefaßt.

Erstaunlicherweise wird bereits nach 48 Stunden ein Tumor/Plasma Verhältnis von 5/1 erreicht. Zu späteren Zeitpunkten wird dieses Verhältnis noch günstiger und erreicht Werte > 200/1 (Tag 5). Die Ursache für dieses günstige pharmakokinetische Verhalten des sFv-huβ-Gluc Fusionsproteins liegt darin, daß nicht am Tumor gebundenes Fusionsprotein hauptsächlich über Rezeptoren für Mannose-6-Phosphat und Galaktose aus dem Plasma und den Normalgeweben durch Internalisation entfernt wird. (Diese Aussage läßt sich dadurch belegen, daß die β-Glucuronidase Werte intrazellulär, z.B. in der Leber, ansteigen).

Wie in Tabelle 5 gezeigt, enthält das sFv-huβ-Gluc größere Mengen an Galaktose und vor allem Mannose, die hauptsächlich für die Anbindung an die jeweiligen Rezeptoren verantwortlich sind. Der so entstandene, über die Kohlenhydratreste des Fusionsproteins gebundene Fusionsprotein-Rezeptorkomplex wird dann durch Internalisation aus dem extrazellulären Kompartiment entfernt.

Dieser hauptsächlich über Galaktose und Mannose vermittelte schnelle Internalisationsmechanismus ist maßgeblich an der vorteilhaften Pharmakokinetik des erfindungsgemäßen Fusionsproteins beteiligt. Diese vorteilhafte Pharmakokinetik des mit Galaktose und vor allem Mannose besetzten Fusionsproteins ermöglicht die i.v. Applikation einer sich extrazellulär verteilenden, hydrophilen Prodrug zu einem relativ frühen Zeitpunkt, ohne eine unspezifische Prodrugaktivierung hervorzurufen. Hierbei ist ein Eliminierungsschritt wie bei Sharma et al. (Brit. J. Cancer, 61, 659, 1990) beschrieben, nicht nötig. Basierend auf den Daten der Tabelle 4 ist die Injektion einer geeigneten Prodrug (S. Adrianomenjanahari et al. 1992, Int. Carbohydrate Symp., Parts A264, 299) schon 3 Tage nach Injektion des sFv-huβ-Gluc Fusionsproteins ohne Erzeugung von signifikanten Nebenwirkungen möglich (Daten nicht gezeigt).

Ein ähnlich vorteilhafter Kohlenhydratbesatz auf Fusionsproteinen ist z.B. auch durch sekretorische Expression des sFv-huβGluc Fusionsprotein in bestimmten Hefestämmen wie Saccharomyces cerevisiae oder Hansenula polymorpha zu erzielen. Diese Organismen sind in der Lage, Fusionsproteine, die entsprechende N-Glykosylierungsstellen besitzen, sehr wirkungsvoll zu mannosylieren (Goochee et al., Biotechnology, 9, 1347-1354, 1991). Solche sekretorisch in Hefezellen exprimierten Fusionsproteine zeigen einen hohen Mannosylierungsgrad und eine dem in BHK-Zellen exprimierten sFv-huβ-Gluc Fusionsprotein vergleichbare günstige Pharmakokinetik (Daten nicht gezeigt). Hierbei wird die Abwesenheit von Galaktose durch den noch höheren Mannosylierungsgrad des Fusionsproteins ausgeglichen (Tabelle 6). Das oben beschriebene sFv-huβ-Gluc Fusionsprotein wurde, wie in Beispiel 9 näher beschrieben, gentechnisch konstruiert und in Hefe exprimiert.

Anstelle der humanen β-Glucuronidase kann man jedoch auch eine andere Glucuronidase mit vorteilhaften Eigenschaften einsetzen. Beispielsweise hat die *E.coli* β-Glucuronidase insbesondere den Vorteil, daß ihre katalytische Aktivität bei pH 7.4 signifikant höher ist als die der humanen β-Glucuronidase. In Beispiel 10 wurde mittels gentechnischer Methoden ein sFv-E. coli β-Gluc Konstrukt hergestellt und in Saccharomyces cerevisiae sekretorisch als funktionell aktives mannosyliertes Fusionsprotein exprimiert. Die pharmakokinetischen Daten sind denen des sFv-huβ-Gluc Moleküls, welches in Hefe bzw. in BHK-zellen exprimiert wurde (Tabelle 4), vergleichbar.

Die Glucuronidasen aus dem Pilz Kobayasia nipponica und aus der Pflanze *Secale cereale* haben z.B. den Vorteil, daß sie auch als Monomere aktiv sind. In Beispiel 11 ist mittels gentechnischer Methoden ein Konstrukt hergestellt worden, welches nach Expression in Saccharomyces cerevisiae ein sFv-B. cereus β-lactamase II Fusionsprotein in vorzugsweise mannosylierter Form ausscheidet.

Dieses Fusionsprotein hat ebenfalls, wie die erfindungsgemäßen Fusionsproteine, auf β-Glucuronidase-Basis eine für die Prodrugaktivierung günstige Pharmakokinetik (Tabelle 4).

Ferner können die erfindungsgemäßen Verbindungen nicht nur in Kombination mit einer Prodrug, sondern auch im Rahmen der gängigen Chemotherapie eingesetzt werden, bei der als Glucuronide metabolisierte und somit inaktivierte Zytostatika durch die applizierten Verbindungen wieder in ihre toxische Form umgewandelt werden können.

Die nachfolgenden Beispiele beschreiben nun die gentechnische Synthese von sFv-huβ-Gluc Fusionsproteinen, sowie den Nachweis der Funktionsfähigkeit.

Ausgangsmaterial waren die Plasmide pABstop 431/26 hum V_{H} und pABstop 431/26 hum VH_{L}. Diese Plasmide enthalten die humanisierte Version des V_{H}- bzw. V_{L}-Gens des anti CEA MAK BW 431/26 (Güssow und Seemann, 1991, Meth. Enzymology, 203, 99-121). Als weiteres Ausgangsmaterial diente das Plasmid pABstop 431/26 V_{H}-huβ-Gluc 1H (EP-A2-0 501 215), das ein V_{H}-Exon, einschließlich der V_{H}-eigenen Signalsequenz, gefolgt von einem CH1-Exon, dem Hinge-Exon eines humanen IgG3 C-Gens und die vollständige cDNA der humanen β-Glucuronidase enthält.

### Beispiel 1:

### Amplification der V_{H} und V_{L} Gene des MAk hum 431/26

Mit den Oligonukleotiden pAB-Back und Linker-Anti (Tab. 2) wird aus pABstop 431V_{H} hum das V_{H}-Gen einschließlich der V_{H}-Gen eigenen Signalsequenz herausamplifiziert (V_{H} 431/26) (Güssow und Seemann, 1991, Meth. Enzymology, 203, 99-121). Mit den Oligonukleotiden Linker-Sense und V_{L(Mut)}-For (Tab. 3) wird aus pABstop 431V_{L} hum das V_{L}-Gen herausamplifiziert (V_{L} 431/26).

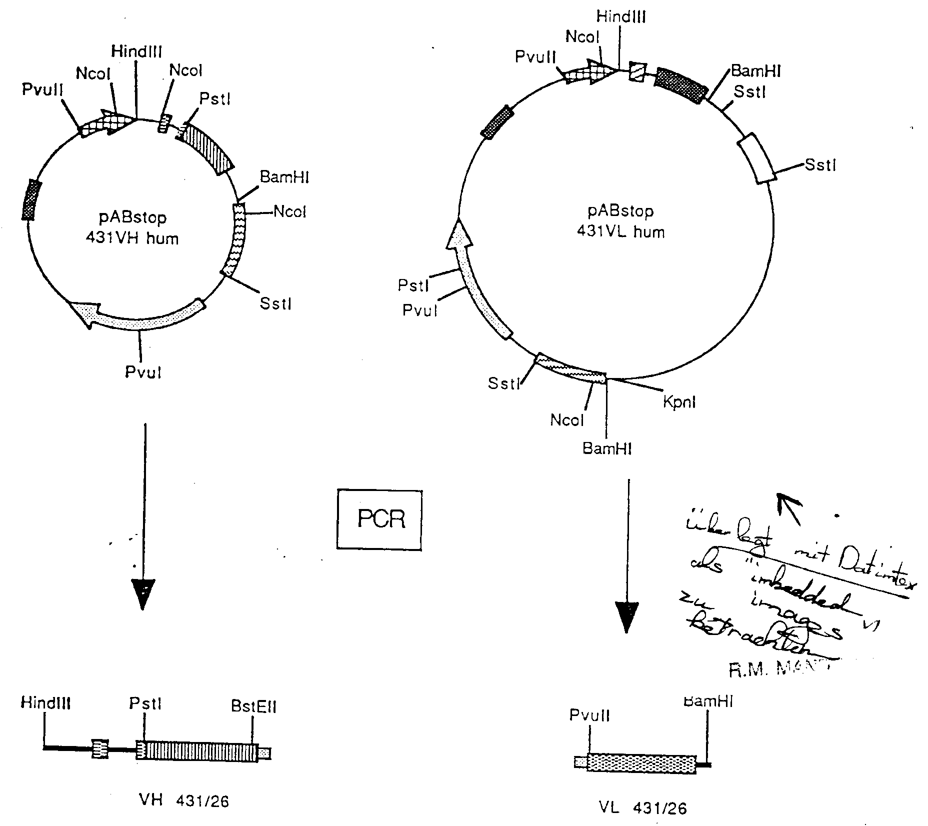
HindIII
NcoI
PvuII
NcoI
HindIII
NcoI
PvuII
PstI
BamHI
SstI
BamHI
NcoI
pABstop
431VH hum
pABstop
431VL hum
SstI
SstI
PstI
PvuI
PvuI
SstI
KpnI
NcoI
BamHI
PCR
überlegt mit Datintex
als "imbedded images"
zu betrachten
R.M. MAN[?]
HindIII PstI BstEII
BamHI
PvuII
VH 431/26
VL 431/26

### Beispiel 2:

### Zusammenfügen der V_{H} 431/26 und V_{L} 431/26 Genfragmente

Die Oligonukleotide Linker-Anti und Linker-Sense sind partiell komplementär zueinander und codieren für einen Polypeptid-Linker, der die V_{H}- und V_{L}-Domäne zu einem sFv-Fragment verknüpfen soll. Um die amplifizierten V_{H}- mit den V_{L}-Fragmenten zu fusionieren, werden sie gereinigt und in einer 10 Zyklen Reaktion wie folgt eingesetzt:

| | |
|---|---|
| H₂O: | 37.5 µl |
| dNTPs (2.5 mM): | 5.0 µl |
| PCR-Puffer (10x): Taq-Polymerase (Perkin-Elmer Corp., Emmeryville, CA) | 5.0 µl |
| (2.5 U/µl): | 0.5 µl |
| 0.5 µg/µl DNA des V_{L}-Frag.: | 1.0 µl |
| 0.5 µg/µl DNA des V_{H}-Frag.: | 1.0 µl |
| PCR-Puffer (10x): 100mM Tris, pH8.3, 500mM KCl, 15 mM MgCl₂, 0.1% (w/v) Gelatin. | |

Die Oberfläche des Reaktionsgemisches wird mit Paraffin versiegelt und anschließend die 10 Zyklen Reaktion in einer PCR-Apparatur mit dem Programm 94°C, 1 min; 55°C, 1 min; 72°C, 2 min, durchgeführt. Danach werden 2,5 pM der flankierenden Primer pAB-Back und V_{L(Mut)}-For zugegeben und weitere 20 Zyklen durchgeführt. Man erhält ein PCR-Fragment, das aus dem V_{H}-Gen besteht, welches über einen Linker mit dem V_{L}-Gen verbunden ist. Vor dem V_{H}-Gen befindet sich noch die V_{H}-Gen eigene Signalsequenz. Durch das Oligonukleotid V_{L(Mut)}-For wird gleichzeitig die letzte Nukleotidbase des V_{L}-Gens, ein C, gegen ein G ausgetauscht. Dieses PCR-Fragment codiert für einen humanisierten Single-Chain-Fv (sFv 431/26).

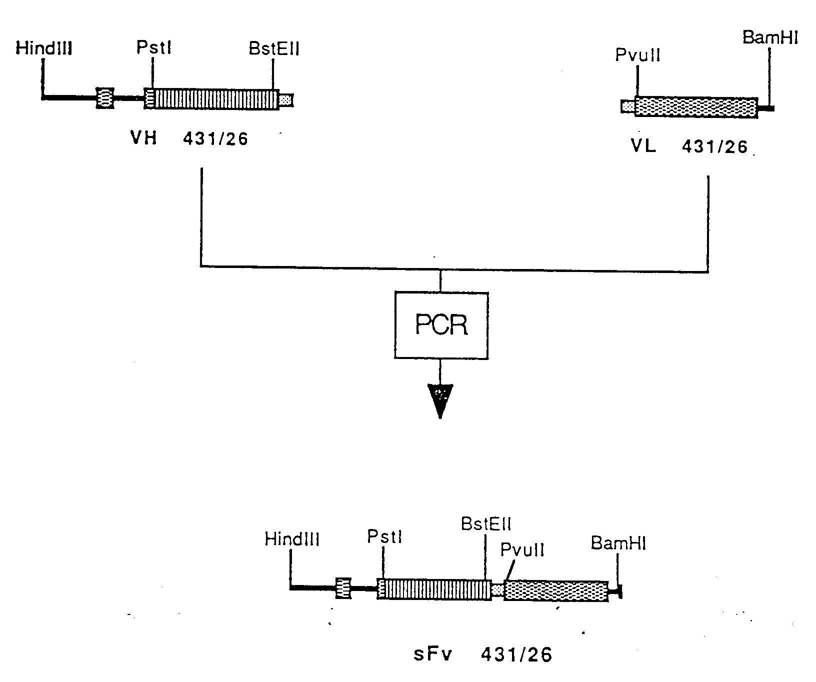
HindIII PstI BstEII
VH 431/26
PvuII BamHI
VL 431/26
PCR
HindIII PstI BstEII PvuII BamHI
sFv 431/26

### Beispiel 3:

### Klonierung des sFv 431/26 Fragmentes in den Expressionsvektor, der das huβ-Glucuronidasegen enthält.

Das sFv-Fragment aus (2) wird mit HindIII und BamHI geschnitten und in den mit HindIII vollständig und mit BglII partiell gespaltenen Vektor pAB 431V_{H} hum/CH1 + 1h/βGlc ligiert. Der Vektor pABstop 431/26V_{H}huβGluc1H enthält ein V_{H}-Exon, einschlieβlich der V_{H}-eigenen Signalsequenz, gefolgt von einem CH1-Exon, dem Hinge-Exon eines humanen IgG3 C-Gens und der vollständigen cDNA der humanen β-Glucuronidase. Es wird der Plasmidklon pMCG-E1 isoliert, der den humanisierten sFv 431/26, ein Hinge-Exon und das Gen für die humane β-Glucuronidase enthält (pMCG-E1).

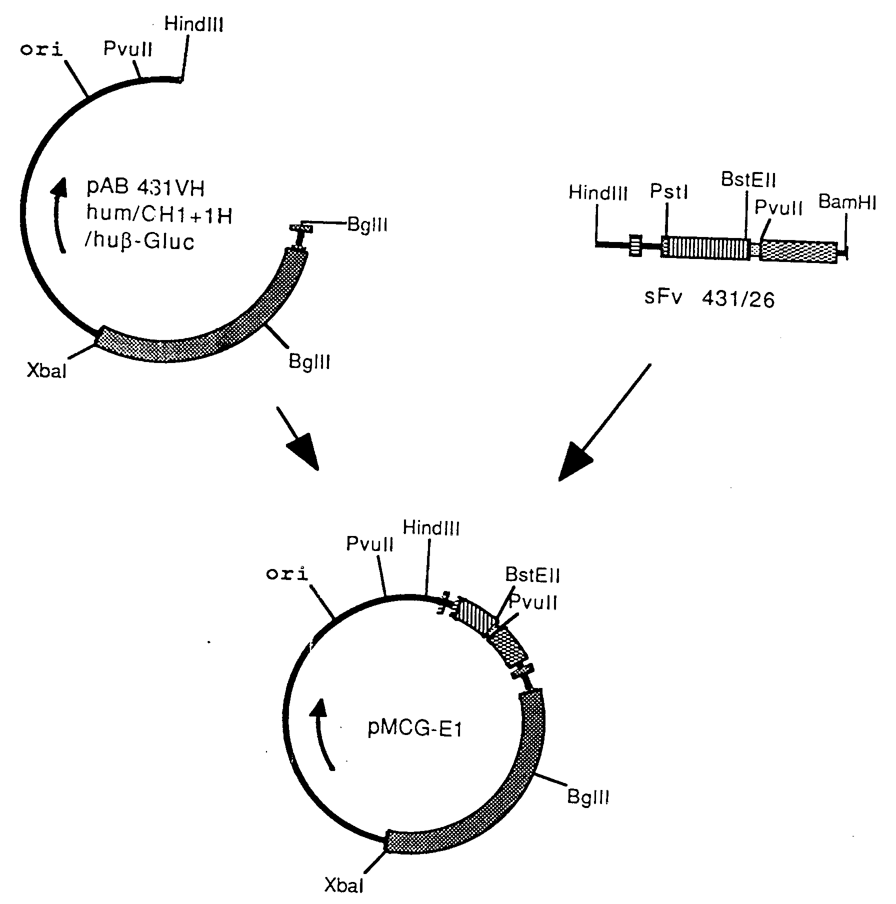
HindIII
ori PvuII
pAB 431VH
hum/CH1+1H
/huβ-Gluc
BglII
XbaI
BglII
HindIII PstI BstEII PvuII BamHI
sFv 431/26
HindIII
PvuII
ori
BstEII
PvuII
pMCG-E1
BglII
XbaI

### Beispiel 4:

### Expression des sFv-huβ-Gluc Fusionsproteins in BHK Zellen.

Der Klon pMCG-E1 wird zusammen mit dem Plasmid pRMH 140, das ein Neomycin-Resistenzgen trägt und dem Plasmid pSV2, das ein Methotrexat-Resistenzgen trägt, in BHK Zellen transfiziert. Daraufhin wird von den BHK Zellen ein Fusionsprotein ausgeprägt, das sowohl die Antigenbindungseigenschaften des MAK BW 431/26hum als auch die enzymatische Aktivität der humanen β-Glucuronidase hat.

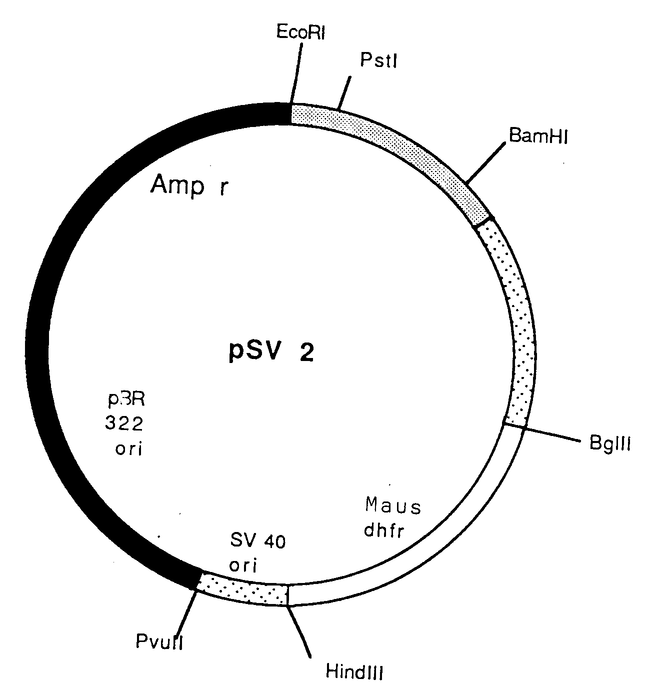
EcoRI
PstI
BamHI
Amp r
pSV 2
BglII
pBR
322
ori
Maus
dhfr
SV 40
ori
PvuII
HindIII

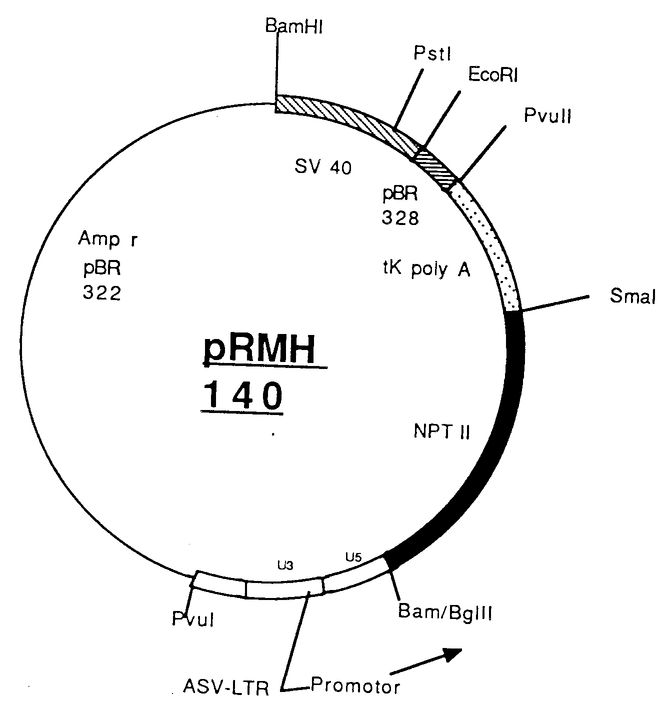
BamHI
PstI
EcoRI
PvuII
SV 40
pBR
328
Amp r
pBR
322
tK poly A
SmaI
pRMH
140
NPT II
U3
U5
PvuI
Bam/BglII
ASV-LTR
Promotor

### Beispiel 5:

### Nachweis der Antigenbindungseigenschaften und der enzymatischen Aktivität des sFv-huβ-Gluc Fusionsproteins.

Die Fähigkeit des sFv-huβ-Gluc Fusionsproteins spezifisch an das durch den 431/26 definierte Epitop auf CEA zu binden und gleichzeitig die enzymatische Aktivität der humanen β-Glucuronidase auszuüben, wurde in einem Spezifitäts-Enzymaktivitätstest gezeigt (EP-A2-0501215). Der Test bestimmt die Freisetzung von 4-Methyl-umbelliferon aus 4-Methyl-umbelliferyl-β-Glucuronid durch den β-Glucuronidase Anteil des Fusionsproteins, nachdem das Fusionsprotein über den sFv-Anteil an ein Antigen gebunden ist. Die ermittelten Fluoreszenzwerte werden als relative Fluoreszenzeinheiten (FE) angegeben. Der Test zeigt eine signifikante Methyl-umbelliferon Freisetzung durch das Fusionsprotein in den mit CEA beschichteten Platten. Dagegen wird durch das Fusionsprotein kein Methyl-umbelliferon in mit PEM (polymorphic epithelial mucin) beschichteten Kontrollplatten freigesetzt.

### Beispiel 6:

### TSK-3000 Gelchromatographie

Von dem über Anti-idiotyp Affinitätschromatographie gereinigten sFv-huβ-Gluc Fusionsprotein wurden 200 ng in 25 µl auf einer TSK Gel G 3000 SW XL Säule (TOSO HAAS Best.Nr. 3.5Wx N3211, 7.8 mm x 300 mm) in einem geeigneten Laufmittel (PBS, pH 7.2, enthaltend 5 g/l Maltose und 4.2 g/l Arginin) mit einer Flußrate von 0.5 ml/min chromatographiert. Die Merck Hitachi HPLC-Anlage (L-4000 UV-Detektor, L-6210 Intelligent Pump, D-2500 Chromato-Integrator) wurde mit ≈ 20 bar betrieben, die optische Dichte des Eluats wurde bei 280 nm bestimmt, und mittels eines LKB 2111 Multisac Fraktionssammlers wurden 0.5 ml Fraktionen gesammelt, die anschließend im Spezifitätsenzymaktivitätstest (SEAT) (EP 0501215 A2, Beispiel J) analysiert wurden. Das Ergebnis dieses Experimentes ist in Abb. 1 gezeigt. Es ist deutlich zu erkennen, daß die Position des durch optische Dichtemessung bei 280 nm detektierbaren Peaks mit dem Peak übereinstimmt, der die Spezifität und Enzymaktivität (SEAT) des Eluats bestimmt. Basierend auf den mittels Pfeilen angedeuteten Molekulargewichtspositionen von Standardproteinen kann gefolgert werden, daß das funktionell aktive sFv-huβ-Gluc Fusionsprotein unter nativen Bedingungen ein ungefähres Molekulargewicht von ≈ 200 kDa hat.

### Beispiel 7:

### Aufarbeitung von Organen/Tumoren zur Fusionsproteinbestimmung

Folgende sequentielle Schritte wurden durchgeführt:
- mit Fusionsprotein bzw. Antikörperenzymkonjugat behandelte Nacktmäuse (CD1), die einen subkutanen Tumor haben, werden retroorbital entblutet und dann getötet
- das Blut wird sofort in ein Eppendorfgefäß gegeben, in dem sich schon 10 µl Liquemin 25000 (Fa. Hoffman-LaRoche AG) befindet
- dann wird 10 min bei 2500 U/min in einer Zentrifuge (Megafuge 1.0, Fa. Heraeus) zentrifugiert
- danach wird das Plasma gewonnen und bis zur Testung eingefroren
- die Organe bzw. der Tumor werden entnommen und gewogen
- dann werden sie mit 2 ml 1 % BSA in PBS, pH 7.2, vollständig homogenisiert
- die Tumorhomogenate werden mit 0.1 N HCl auf pH 4.2 eingestellt (die Probe darf nicht übertitriert werden, da die β-Glucuronidase bei pH < 3.8 inaktiviert wird!)
- alle Homogenate werden 30 min bei 16000 g zentrifugiert
- der klare Überstand wird abgenommen
- die Tumorüberstände werden mit 0.1 N NaOH neutralisiert
- die Überstände und das Plasma können nun in immunologischen Tests quantifiziert werden.

### Beispiel 8:

### Triple-Determinanten-Test

Die Testung läuft folgendermaßen ab:
- pro Loch einer Mikrotitrationsplatte (Polystyrol U-Form, Typ B, Fa. Nunc, Best.Nr. 4-60445) werden 75 µl eines mit 2 µg/ml in PBS, pH 7.2, verdünnten Maus-anti-huβ-Gluc Antikörpers (MAk 2118/157 Behringwerke) gegeben
- die Mikrotitrationsplatten werden abgedeckt und über Nacht bei R.T. inkubiert
- anschließend werden die Mikrotitrationsplatten 3x mit 250 µl 0.05 M Tris-Citrat-Puffer, pH 7.4, pro Loch gewaschen
- diese so beschichteten Mikrotitrationsplatten werden pro Loch mit je 250 µl Blocklösung (1 % Casein in PBS, pH 7.2) für 30' bei R.T. inkubiert (Blockierung unspezifischer Bindungsstellen) (nicht benötigte beschichtete Mikrotitrationsplatten werden 24 Stunden bei R.T. getrocknet und dann zusammen mit Trockenpatronen zur Langzeitlagerung in beschichtete Aluminiumbeutel eingeschweißt)
- während der Blockierung wird in einer unbehandelten 96 Loch U-Boden Mikrotiterplatte (Polystyrol, Fa. Renner, Best.Nr. 12058) 10 Proben + 2 Positivkontrollen + 1 Negativkontrolle in 1 % Casein in PBS, pH 7.2, 1:2 in 8 Stufen ausverdünnt (ausgehend von 150 µl Probe werden 75 µl Probe in 75 µl Casein-Vorlage pipettiert usw.)
- die Blocklösung wird von der mit anti-huβ-Gluc Antikörpern beschichteten Mikrotitrationsplatte abgesaugt, 50 µl der verdünnten Proben werden pro Loch von der Verdünnungsplatte auf die Testplatte übertragen und 30 min bei R.T. inkubiert
- während der Probeninkubation wird das ABC-AP Reagenz (Fa. Vectastain, Best.Nr. AK-5000) angesetzt: 2 Tropfen Reagenz A (Avidin DH) in 10 ml 1 % Casein in PBS, pH 7.2, gut mischen und 2 Tropfen Reagenz B (Biotinylierte alkalische Phosphatase) zugeben, gut mischen. (Die ABC-AP Lösung muß mindestens 30' vor Gebrauch angesetzt werden.)
- die Testplatte wird 3 mal mit ELISA Waschpuffer (Behringwerke, Best.Nr. OSEW 96) gewaschen
- pro Loch werden 50 µl Biotin markiertes Nachweisantikörpergemisch (1 + 1 Gemisch aus Maus Anti 431/26 Antikörper (MAk 2064/353, Behringwerke) und Maus anti CEA Antikörper (MAK 250/183, Behringwerke) mit einer Konzentration von je 5 µg/ml verdünnt in 1 % Casein in PBS, pH 7.2, Endkonzentration 2.5 µg/ml je Antikörper) gegeben
- die Testplatte wird 3 mal mit ELISA Waschpuffer gewaschen
- pro Loch werden 50 µl der vorbereiteten ABC-AP Lösung gegeben und 30 min bei R.T. inkubiert
- während der ABC-AP Inkubation wird das Substrat angesetzt (für jeden Test frisches Substrat: 1 mM 4-Methylumbelliferyl Phosphat, Best.Nr. M-8883, Fa. Sigma, in 0.5 M Tris + 0.01 % MgCl, pH 9.6)
- die Testplatte wird 7 mal mit ELISA Waschpuffer gewaschen
- pro Loch werden 50 µl Substrat aufgetragen, die Testplatte abgedeckt und 2 h bei 37° C inkubiert
- danach wird zu jedem Loch 150 µl Stopplösung (0.2 M Glycin + 0.2 % SDS, pH 11.7) hinzugegeben
- die fluorometrische Auswertung erfolgt im Fluoroscan II (ICN Biomedicals, Kat.Nr. 78-611-00) bei einer Anregungswellenlänge von 355 nm und einer Ausstrahlungswellenlänge von 460 nm
- anhand der Fluoreszenzwerte der im identischen Experiment mitgeführten Positivkontrolle (Verdünnungsreihe mit gereinigtem sFv-huβ-Gluc gemischt mit CEA 5 µg/ml als Eichkurve) wird die unbekannte Konzentration von Fusionsprotein in der Probe bestimmt.

### Beispiel 9:

### Expression des sFv-huβ-Glucuronidase Fusionsproteins in Hefe.

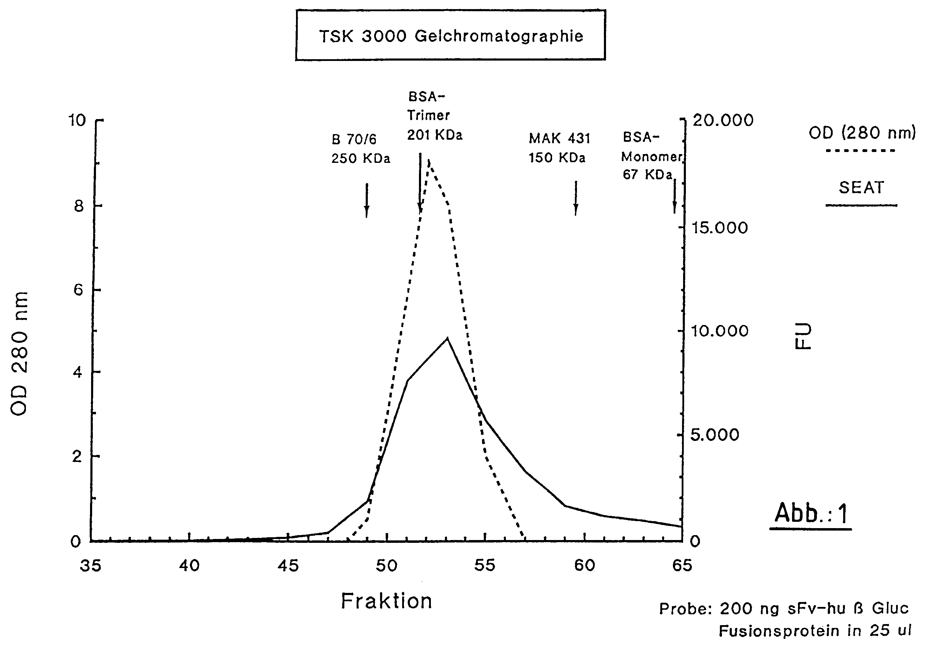

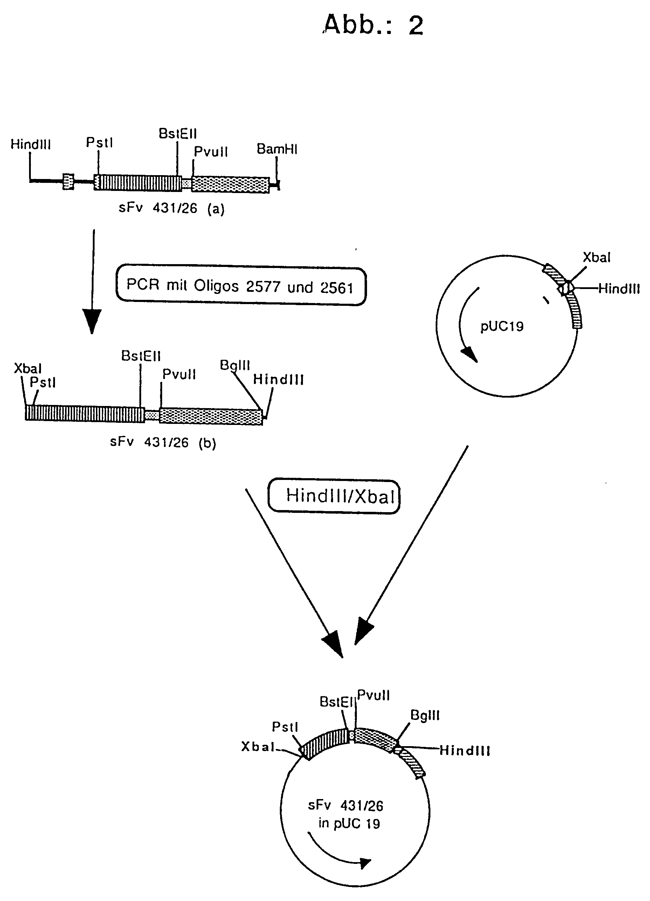

Der Single-chain-Fv (sFv) aus Beispiel 2 wird mit den Oligos 2577 und 2561 (Tabelle 7) amplifiziert und in den mit XbaI/HindIII verdauten pUC19 Vektor kloniert (Abb. 2).

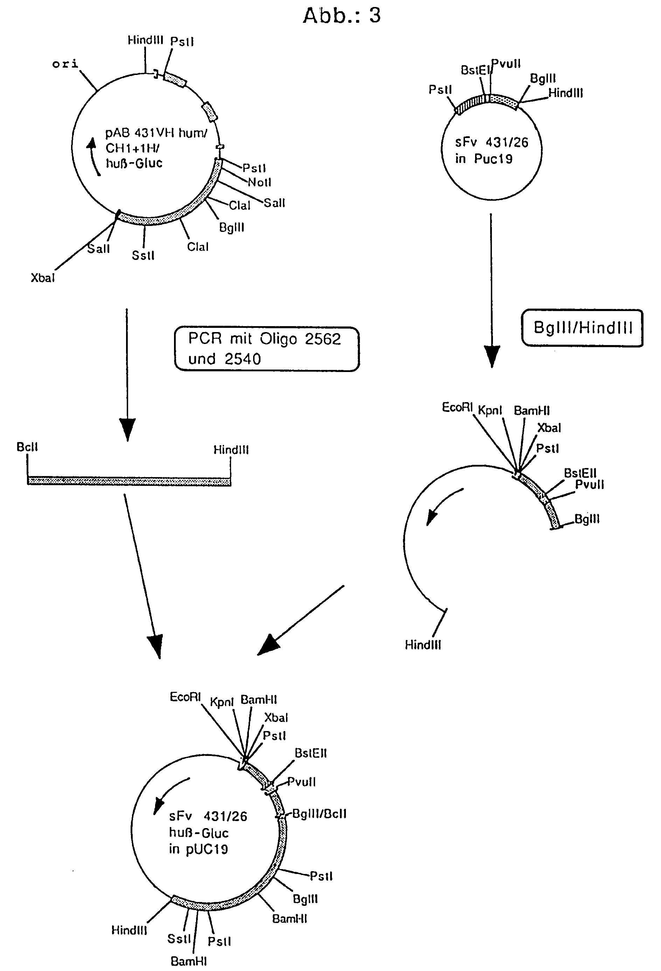

Das humane β-Glucuronidase Gen wird mit den Oligos 2562 und 2540 (Tabelle 8) aus dem Plasmid pAB 431/26 V_{H}hum/CH1 + 1H/huβ-Gluc (Beispiel 3) amplifiziert und in das mit BglII/HindIII geschnittene Plasmid sFv 431/26 in pUC19 (Abb. 2) ligiert (Abb. 3).

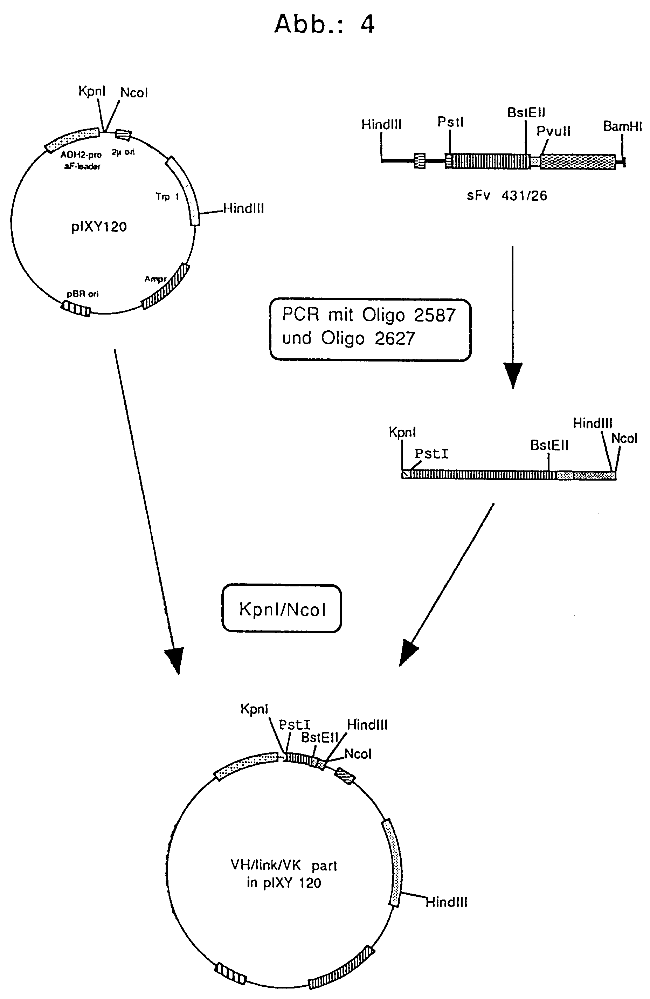

Ein KpnI/NcoI-Fragment wird mit den Oligos 2587 und 2627 (Tabelle 9) aus dem sFv 431/26 amplifiziert und in den mit KpnI/NcoI verdauten Hefe-Expressionsvektor pIXY 120 kloniert (Abb. 4).

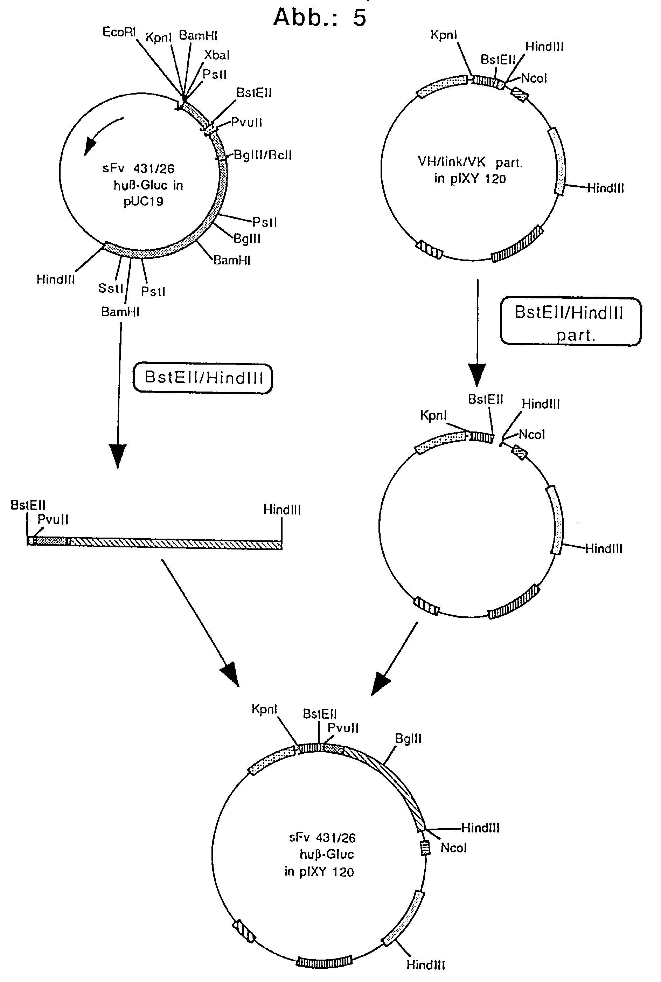

Das BstEII/HindIII Fragment aus dem Plasmid sFv 431/26 huβ-Gluc in pUC19 (Abb. 3) wird in den mit BstEII/partiell HindIII verdauten Vektor pIXY 120 ligiert, der das V_{H}-Gen, den Linker sowie einen Teil des V_{L}-Gens trägt (V_{H}/link/V_{K} part. in pIXY 120) (Abb. 5).

Das entstandene Plasmid sFv 431/26 huβ-Gluc in pIXY 120 wird in Saccharomyces cerevisiae transformiert und das Fusionsprotein ausgeprägt.

### Beispiel 10:

### Expression des sFv-E.coli-β-Glucuronidase Fusionsproteins in Hefe.

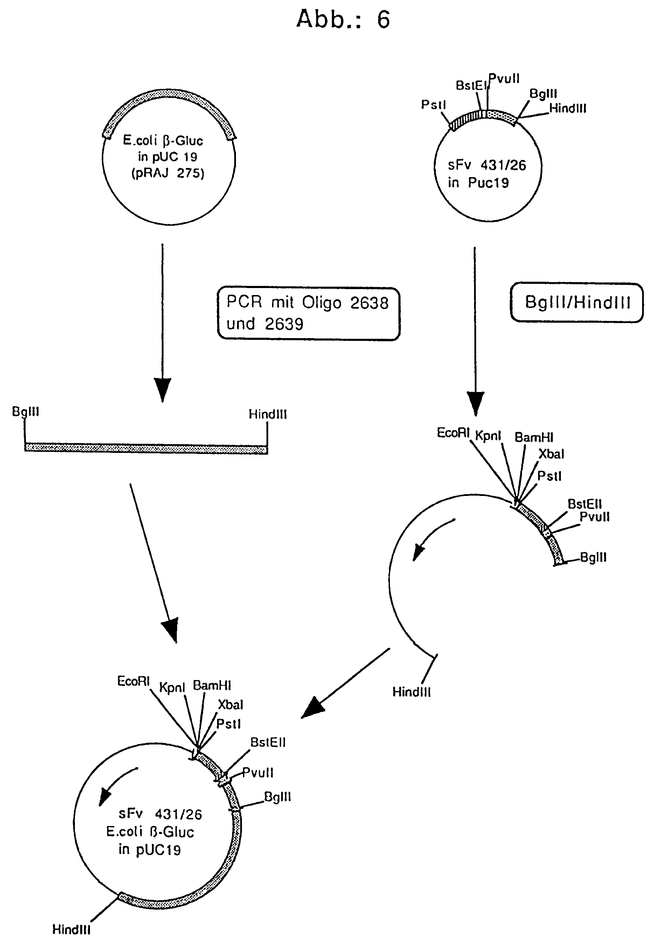

Das E.coli β-Glucuronidase Gen wird aus pRAJ 275 (Jefferson et al. Proc. Natl. Acad. Sci, USA, 83: 8447-8451, 1986) mit den Oligos 2638 und 2639 (Tabelle 10) amplifiziert und in den mit BglII/ HindIII geschnittenen sFv 431/26 in pUC19 (Beispiel 9, Abb. 2) ligiert (Abb. 6).

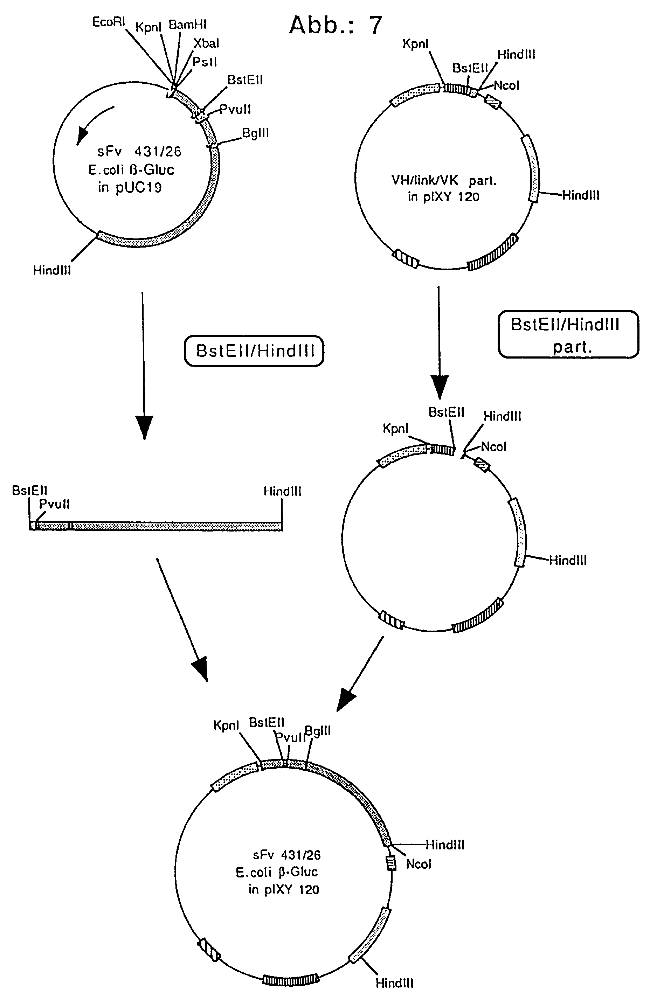

Ein BstEII/HindIII Fragment aus sFv 431/26 E.coli β-Gluc in pUC19 wird in den mit BstEII/HindIII partiell verdauten Vektor V_{H}/link/V_{K}part in pIXY 120 (Beispiel 9, Abb. 4) kloniert (Abb. 7).

Das Plasmid sFv 431/26 E.coli β-Gluc in pIXY 120 wird in Saccharomyces cerevisiae transformiert und das Fusionsprotein ausgeprägt.

### Beispiel 11:

### Expression des sFv-β-lactamase Fusionsproteins in Hefe.

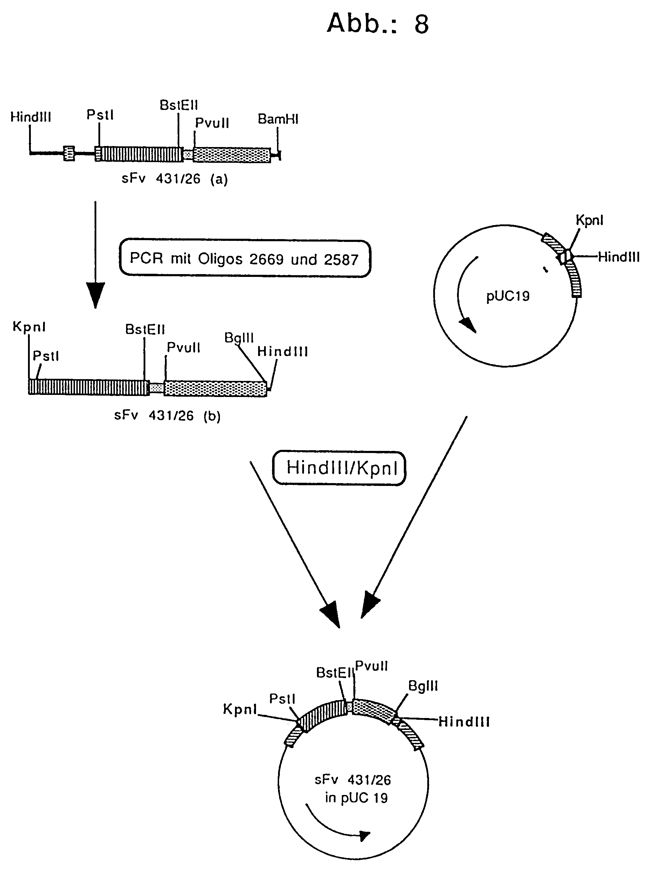

Der Single-chain-Fv (sFv) aus Beispiel 2 wird mit den Oligos 2587 und 2669 (Tabelle 11) amplifiziert und in den mit KpNI/HindIII verdauten pUC19 Vektor ligiert (Abb. 8).

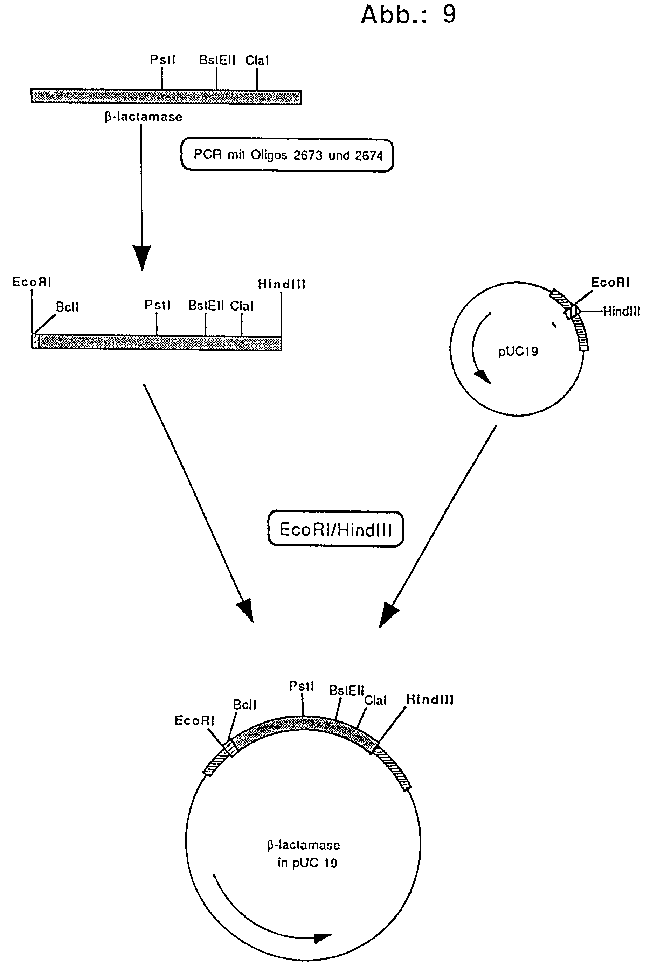

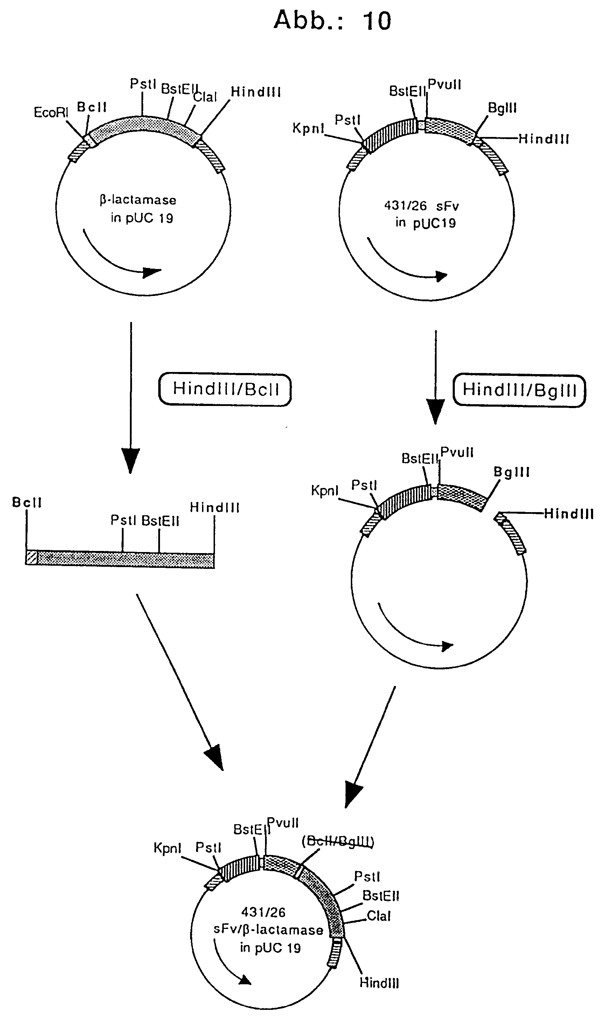

Das β-lactamase II Gen (Hussain et al., J. Bacteriol. 164: 223-229, 1985) wird mit den Oligos 2673 und 2674 (Tabelle 11) aus Gesamt-DNA von Bacillus cereus amplifiziert und in den mit EcoRI/ HindIII verdauten pUC19 Vektor ligiert (Abb. 9). Ein BclI/ HindIII Fragment des β-lactamase Gens wird in den mit BglII/ HindIII geschnittenen sFv 431/26 in pUC19 ligiert (Abb. 10).

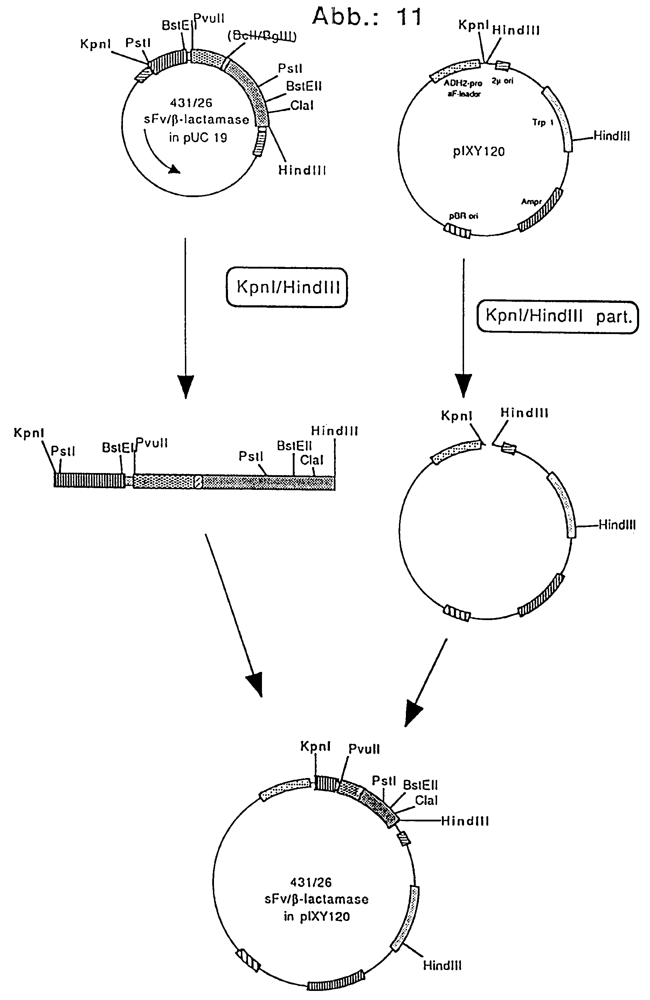

Das KpnI/HindIII sFv-β-lactamase Fragment wird in den mit KpnI/partiell HindIII verdauten pIXY 120 ligiert (Abb. 11). Das Plasmid wird in Saccharomyces cerevisiae transformiert und ein Fusionsprotein ausgeprägt, das sowohl die Antigenbindungseigenschaften des MAk 431/26 als auch die enzymatische Aktivität der β-lactamase von Bacillus cereus trägt.

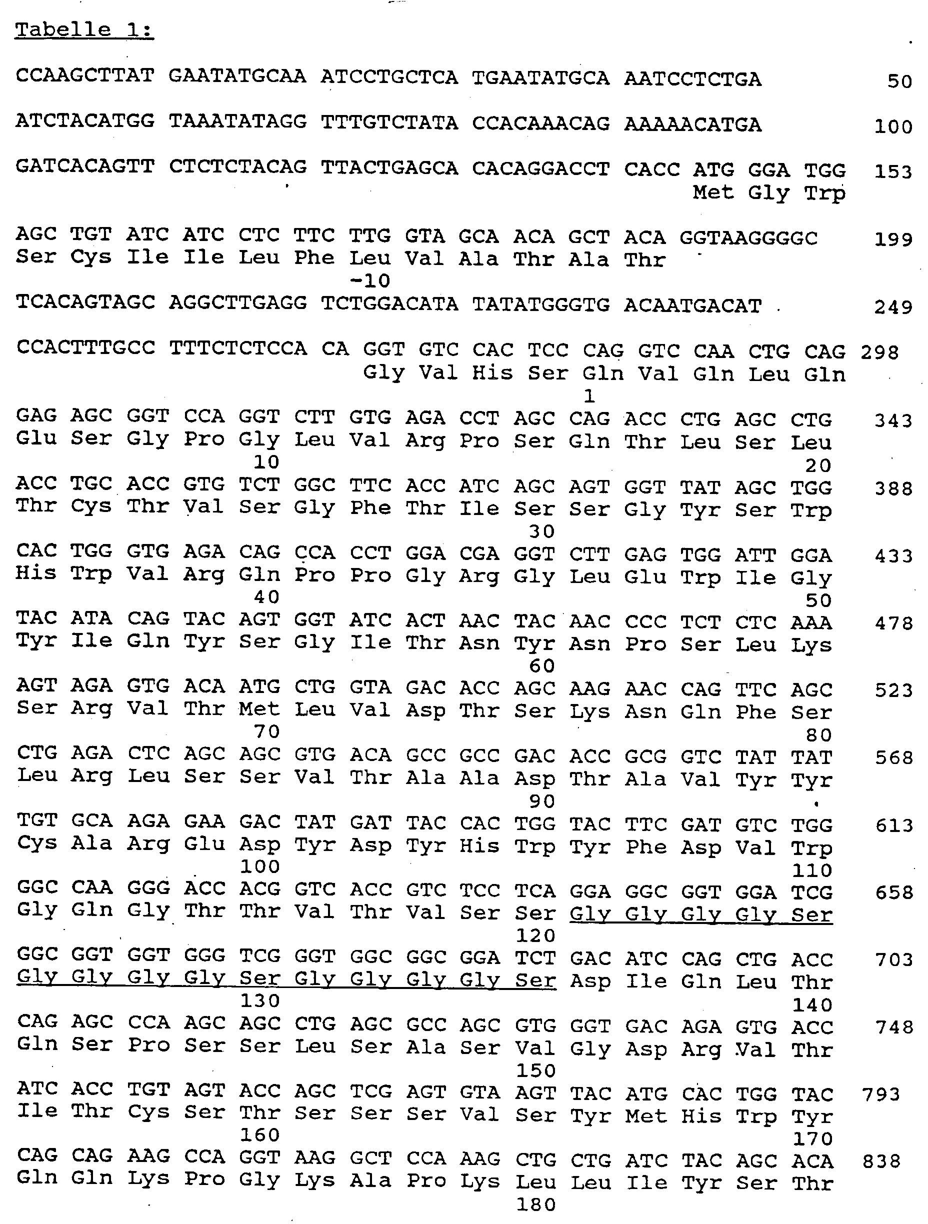

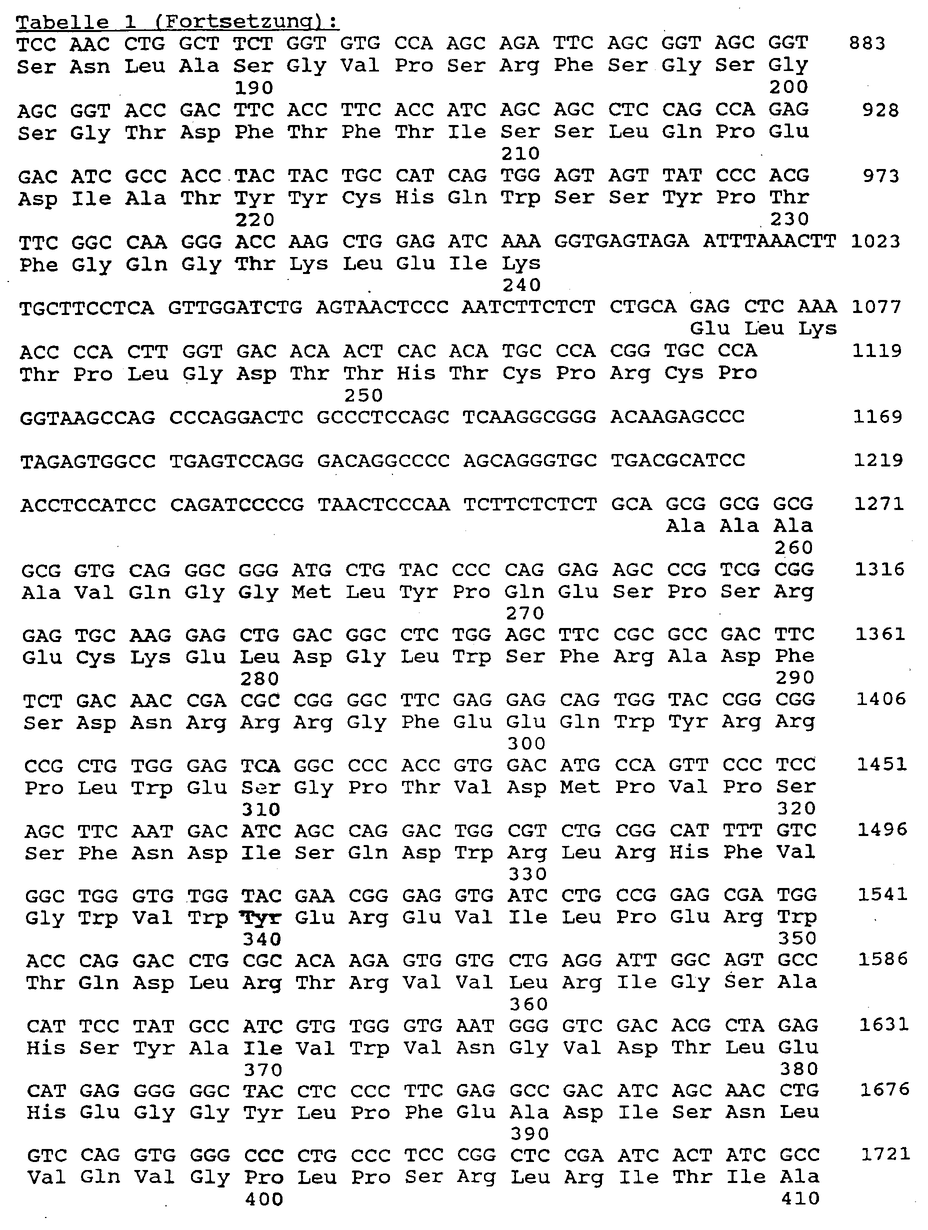

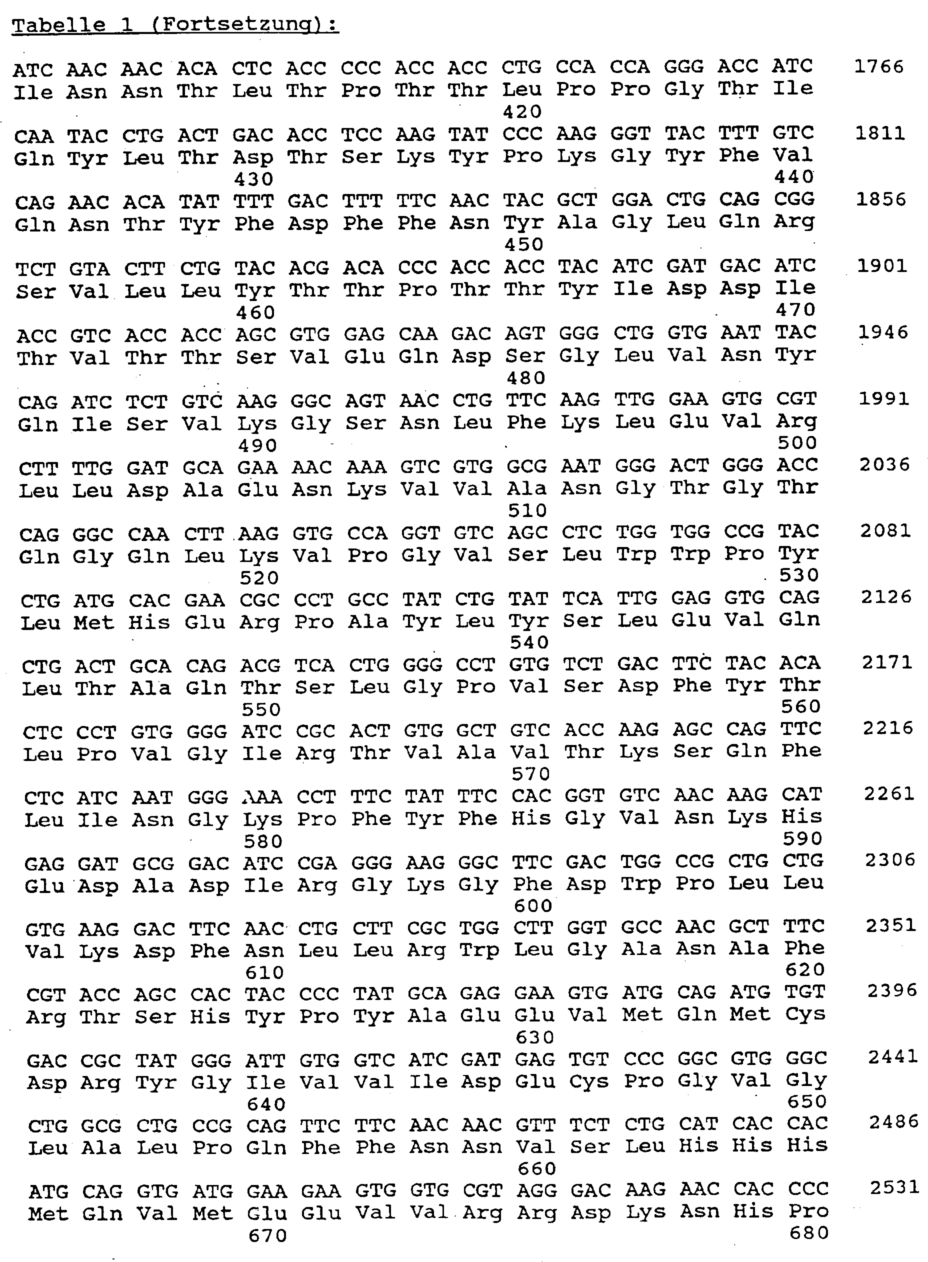

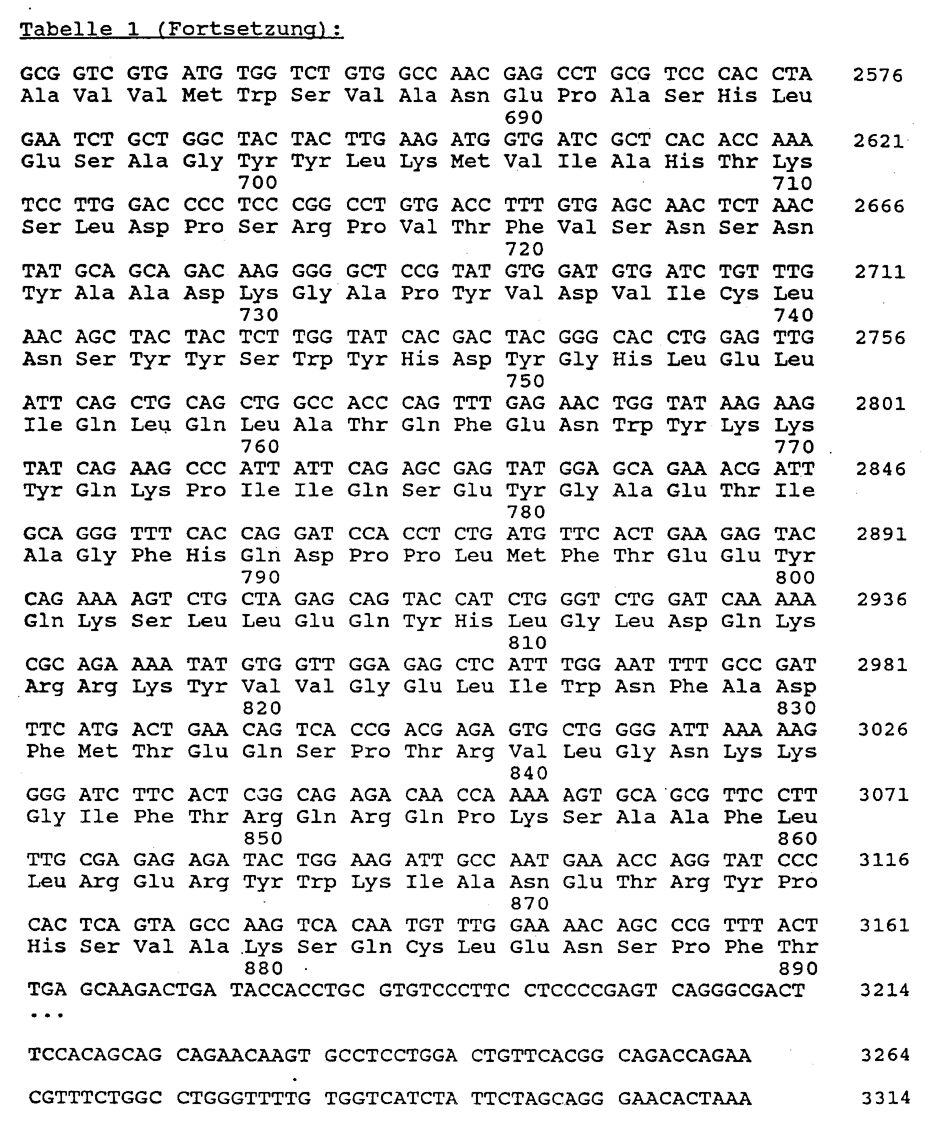

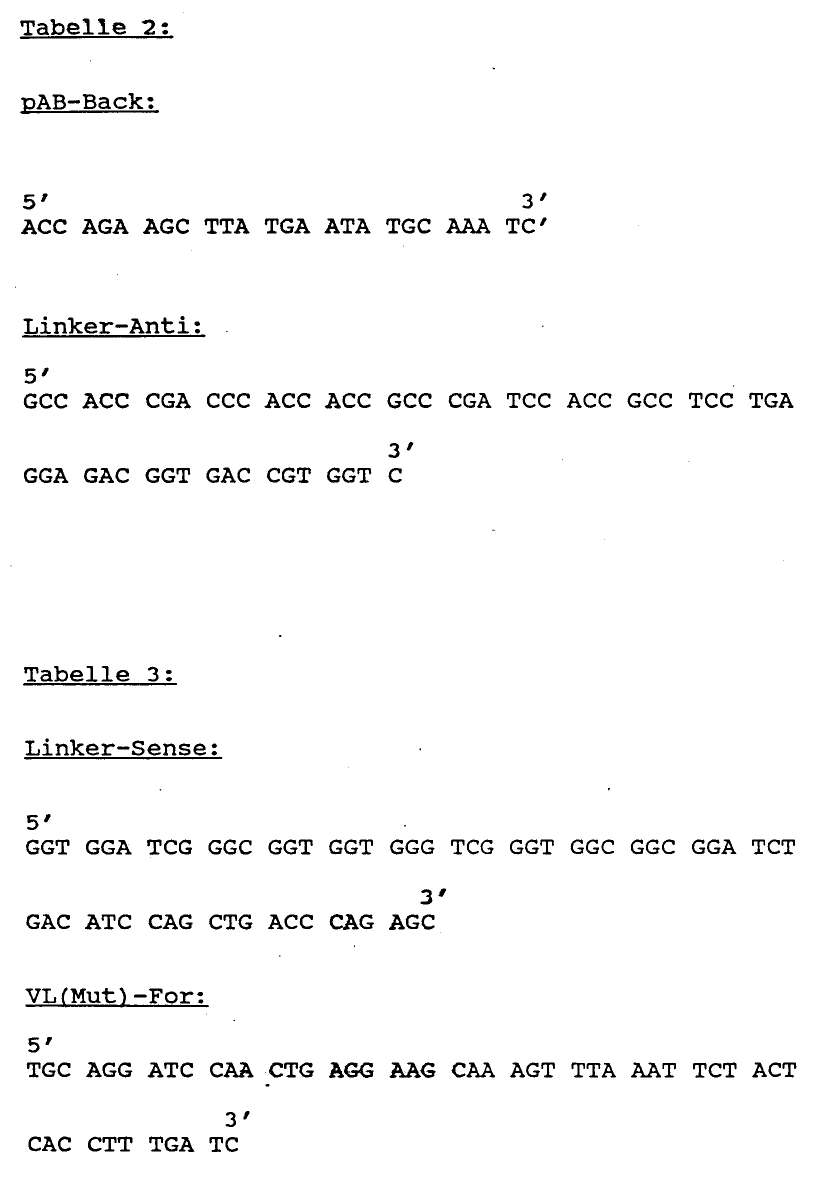

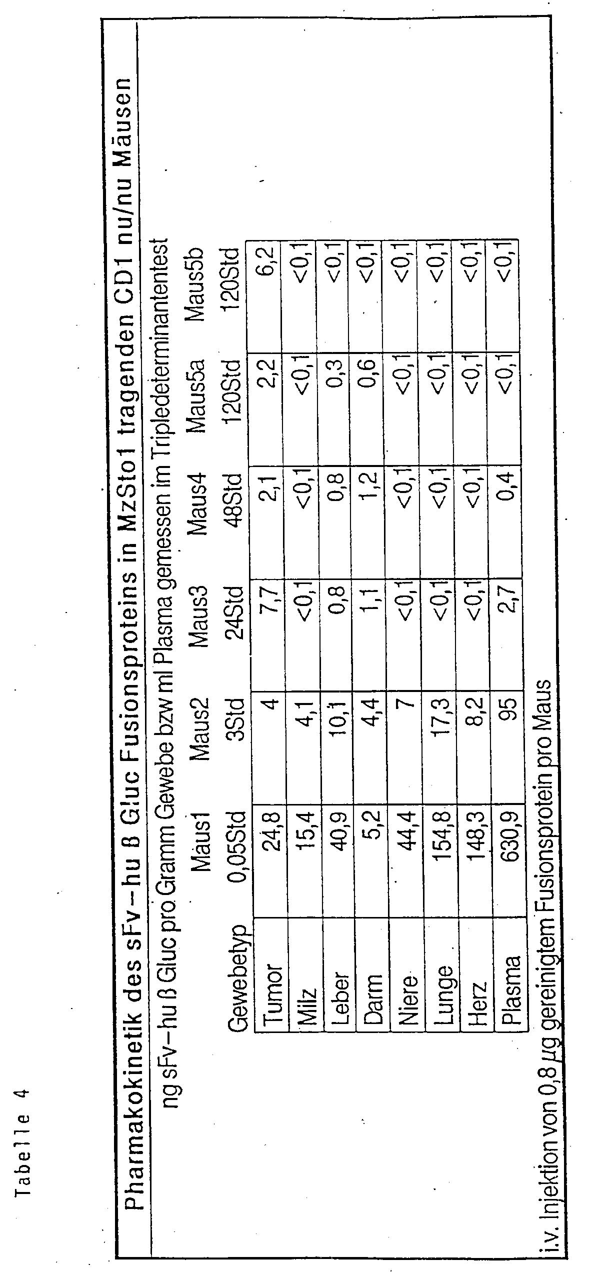

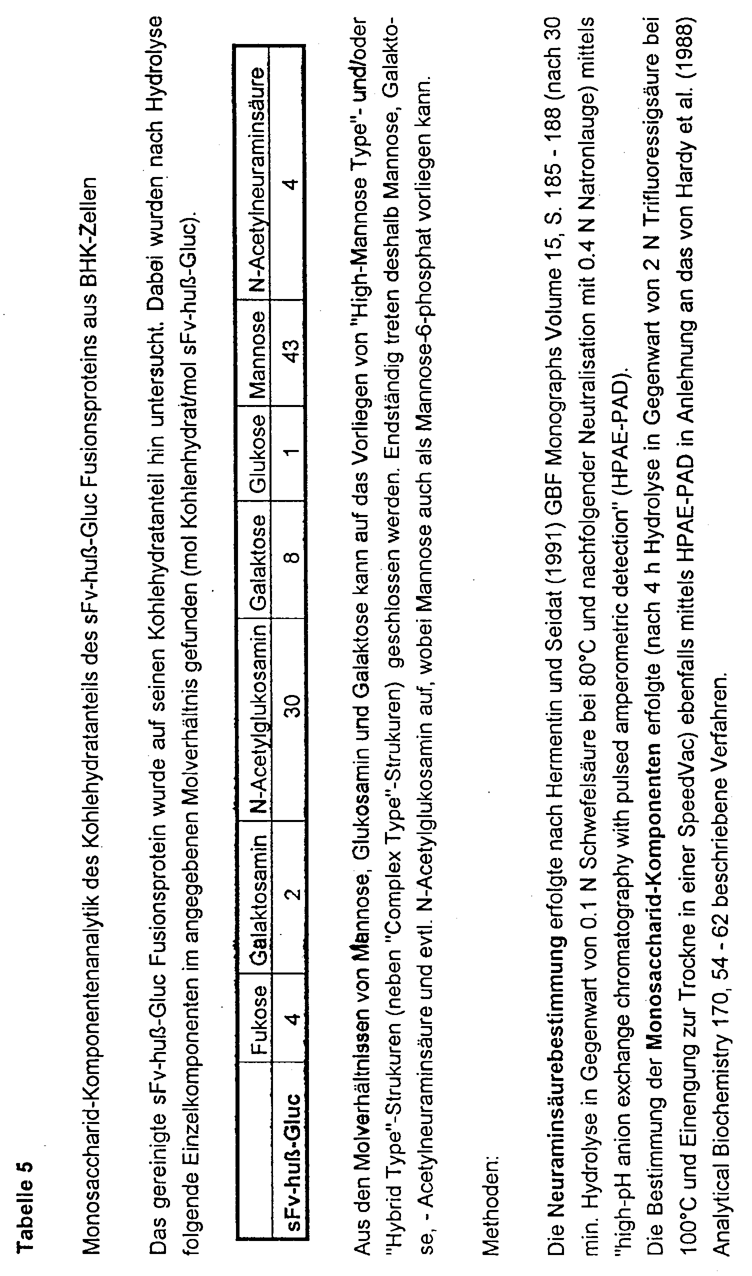

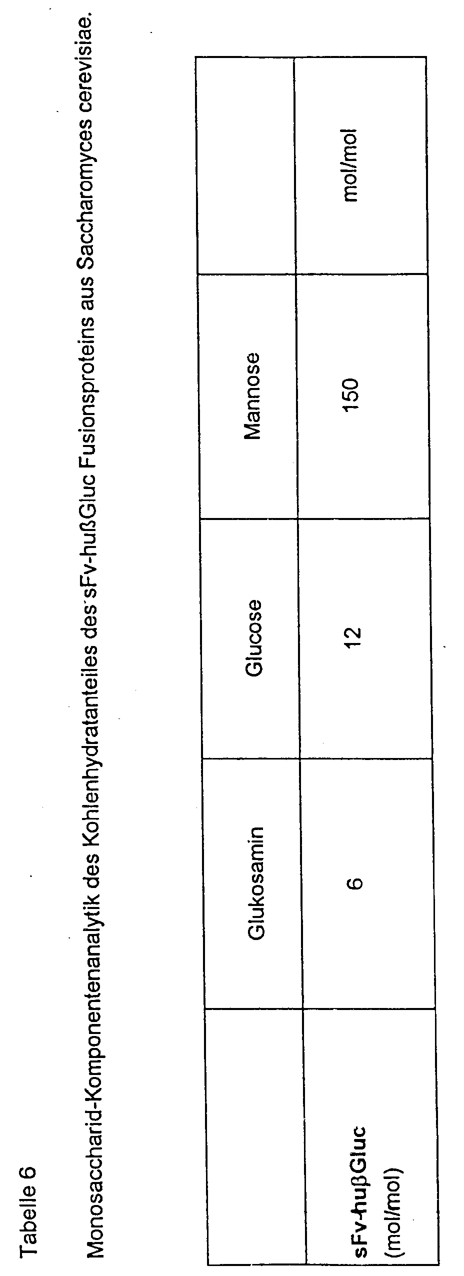

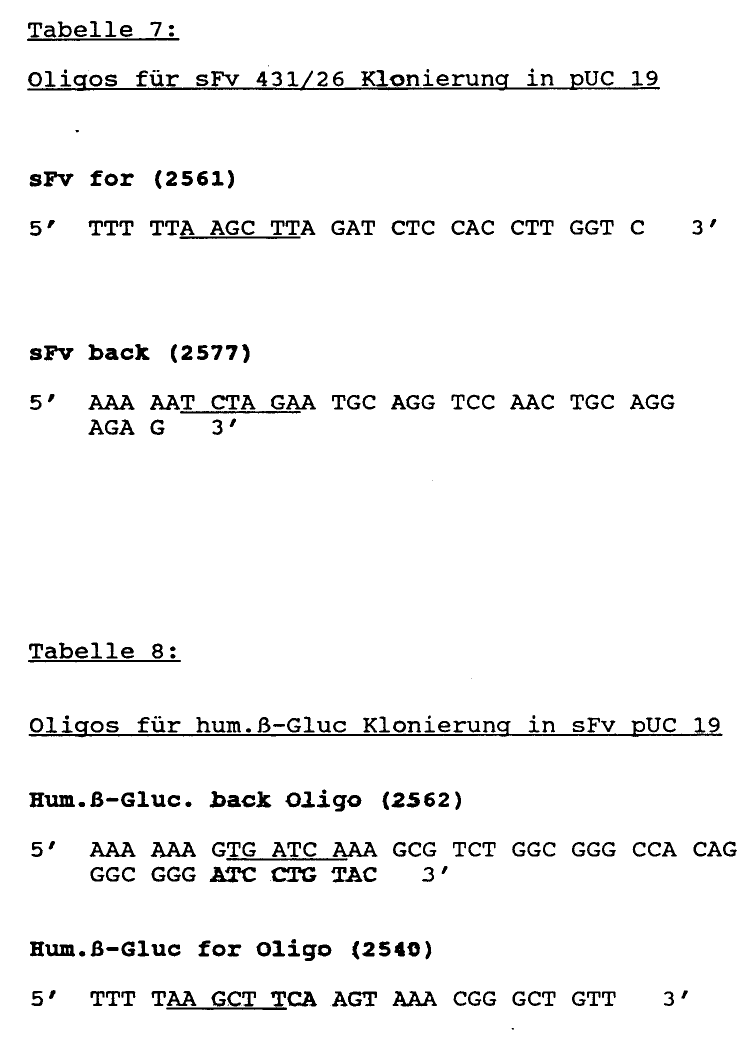

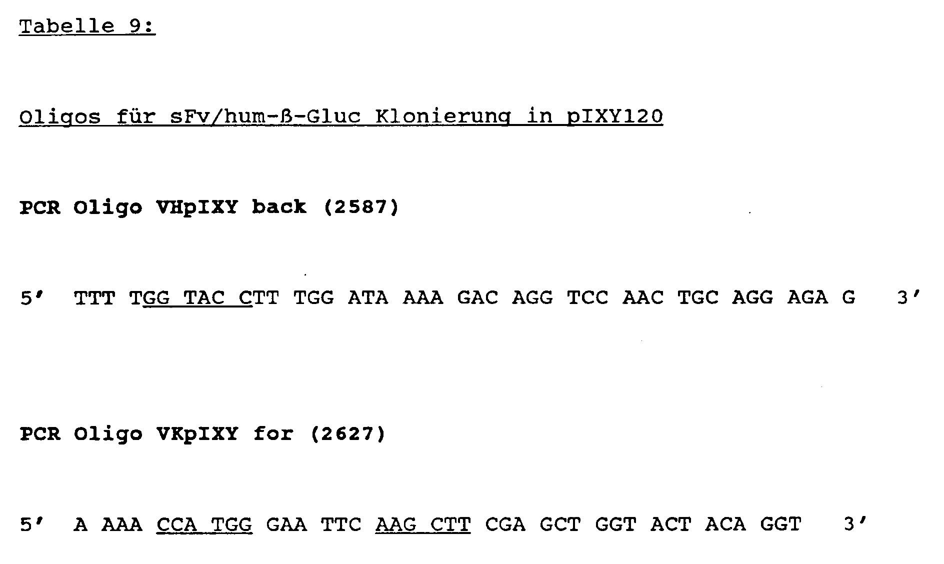

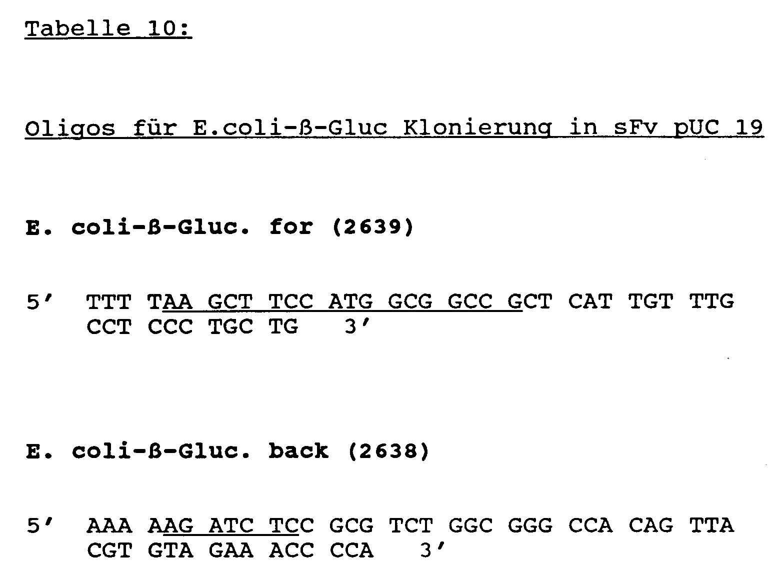

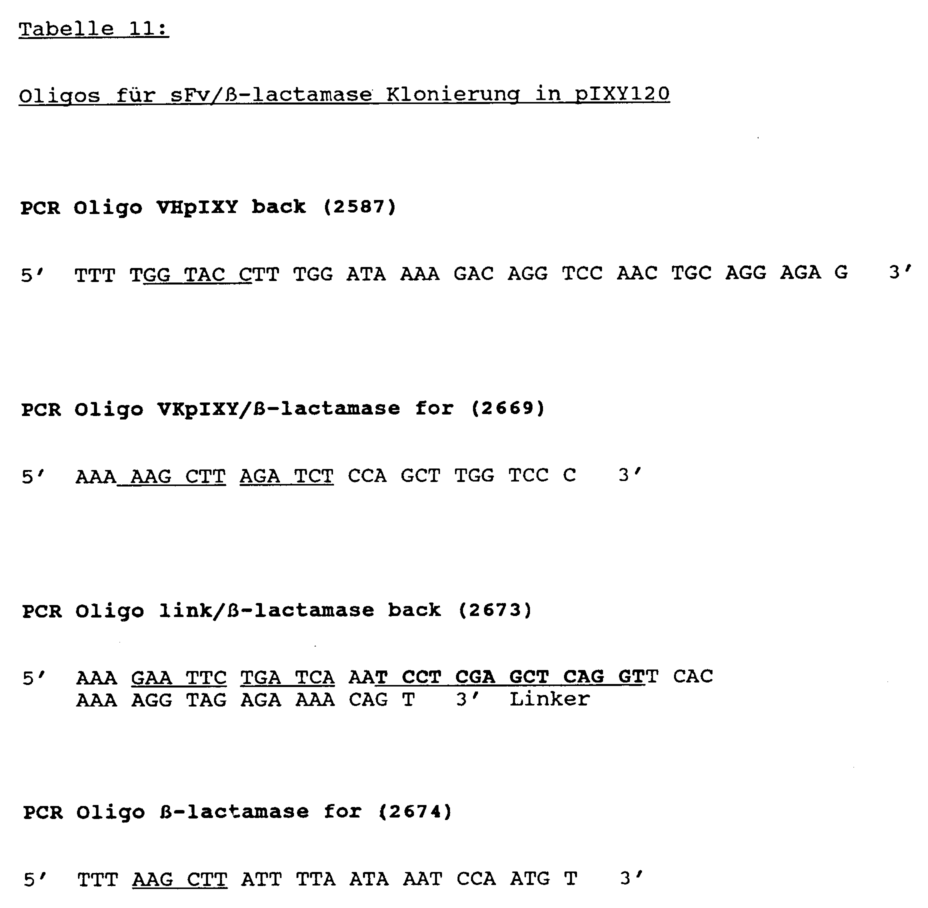

## Patentansprüche

1. Verbindung, enthaltend eine Antigenbinderegion, welche an mindestens ein prodrug-aktivierendes Enzym gebunden ist, dadurch gekennzeichnet, daß die Antigenbinderegion aus einer einzigen Polypeptidkette besteht.

2. Verbindung nach Anspruch 1, dadurch gekennzeichnet, daß die Verbindung kovalent gebundene Kohlenhydrate trägt.

3. Verbindung nach Anspruch 1 und 2, dadurch gekennzeichnet, daß die Antigenbinderegion eine variable Domäne einer schweren Antikörperkette und eine variable Domäne einer leichten Antikörperkette enthält (sFv-Fragment).

4. Verbindung nach einem der Ansprüche 1 oder 2, dadurch gekennzeichnet, daß die Antigenbinderegion an ein tumorassoziiertes Antigen (TAA) bindet.

5. Verbindung nach Anspruch 3, dadurch gekennzeichnt, daß das TAA ein N-CAM, PEM, EGF-R, Sialyl-Le^{a}, Sialyl-Le^{x}, TFβ, GICA, GD₃, GD₂, TAG72, CA125, das durch den MAk L6 definierte 24-25 kDa Glycoprotein oder CEA, vorzugsweise ein CEA ist.

6. Verbindung nach einem der Ansprüche 1 bis 5, dadurch gekennzeichnet, daß das Enzym eine Lactamase, vorzugsweise eine Bacillus cereus II β-lactamase, Pyroglutamat-Aminopeptidase, D-Aminopeptidase, Oxidase, Peroxidase, Phosphatase, Hydroxynitrillyase, Protease, Esterase, Carboxypeptidase, vorzugsweise eine Carboxypeptidase G2 aus Pseudomonas oder Glycosidase ist.

7. Verbindung nach Anspruch 6, dadurch gekennzeichnet, daß das Enzym eine β-Glucuronidase, vorzugsweise eine *E.coli, Kobayasia nipponica, Secale cereale* oder humane β-Glucuronidase ist.

8. Verbindung nach einem der Ansprüche 1 bis 7, dadurch gekennzeichnet, daß die Antigenbinderegion über einen Peptidlinker mit dem Enzym verbunden ist.

9. Verbindung nach einem der Ansprüche 1 bis 8, dadurch gekennzeichnet, daß die Glycosylierung entweder mittels chemischer Methoden oder durch Auswahl geeigneter Expressionssysteme erfolgt.

10. Verbindung nach Anspruch 1-9, dadurch gekennzeichnet, daß sie sekretorisch in Saccharomyces cerevisiae bzw. vorteilhafter in Hansenula polymorpha exprimiert wird.

11. Verbindung nach Anspruch 1-9, dadurch gekennzeichnet, daß sie in E. coli exprimiert wird und anschließend chemisch glycosyliert, vorzugsweise galaktosyliert und/oder mannosyliert wird.

12. Verbindung nach Anspruch 1-9, dadurch gekennzeichnet, daß das sFv β-lactamase Fusionsprotein, welches periplasmatisch in E. coli exprimiert wurde, chemisch glycosyliert, vorzugsweise galaktosyliert und/oder mannosyliert ist.

13. Verbindung nach Anspruch 1-9, dadurch gekennzeichnet, daß das sFv β-lactamase Fusionsprotein sekretorisch in Saccharomyces cerevisiae bzw. Hansenula polymorpha exprimiert ist.

14. Nukleinsäure, kodierend für eine Verbindung nach einem der Ansprüche 1 bis 8.

15. Nukleinsäure nach Anspruch 14, kodierend für ein humanisiertes sFv-Fragment gegen CEA und eine humane β-Glucuronidase.

16. Nukleinsäure nach Anspruch 14 mit der Sequenz gemäß Tab. 1.

17. Vektor, enthaltend eine Nukleinsäure nach einem der Ansprüche 14 bis 16.

18. Wirtszelle, enthaltend eine Nukleinsäure nach einem der Ansprüche 14 bis 16 oder einen Vektor nach Anspruch 17.

19. Wirtszelle nach Anspruch 18, dadurch gekennzeichnet, daß sie eine BHK-, CHO-, COS-, HeLa-, Insekten-, Tabakpflanzen-, Hefe- oder *E.coli*-Zelle ist.

20. Transgene Säugetiere mit Ausnahme von Mensch, enthaltend eine DNA nach einem der Ansprüche 14 bis 16 oder einen Vektor nach Anspruch 17.

21. Verfahren zur Herstellung einer Verbindung nach Anspruch 1 bis 8, dadurch gekennzeichnet, daß
a) eine Nukleinsäure nach einem der Ansprüche 14 bis 16 oder ein Vektor nach Anspruch 17 in eine Wirtszelle eingebracht wird,
b) die Wirtszelle kultiviert und
c) die Verbindung isoliert wird.

22. Verfahren zur Herstellung einer Verbindung nach Anspruch 1 bis 8, dadurch gekennzeichnet, daß
a) eine Wirtszelle nach Anspruch 18 oder 19 kultiviert und
b) die Verbindung isoliert wird.

23. Verwendung der Verbindung nach Anspruch 1 bis 13 zur Herstellung eines Arzneimittels oder eines Diagnostikums.

24. Verwendung der Verbindung nach Anspruch 1 bis 13 zur Herstellung eines Arzneimittels zur Behandlung von Krebs.

25. Arzneimittel, enthaltend eine Verbindung nach Anspruch 1 bis 13.

26. Diagnostikum, enthaltend eine Verbindung nach Anspruch 1 bis 13.

## Claims

1. A compound containing an antigen binding region which is bound to at least one prodrug-activating enzyme, wherein the antigen binding region is composed of a single polypeptide chain.

2. A compound as claimed in claim 1, wherein the compound carries covalently bonded carbohydrates.

3. A compound as claimed in claim 1 and 2, wherein the antigen binding region contains a variable domain of a heavy antibody chain and a variable domain of a light antibody chain (sFv fragment).

4. A compound as claimed in either of claims 1 or 2, wherein the antigen binding region binds to a tumor-associated antigen (TAA).

5. A compound as claimed in claim 3, wherein the TAA is an N-CAM, PEM, EGF-R, Sialyl-Le^{a}, Sialyl-Le^{x}, TFβ, GICA, GD₃, GD₂, TAG72, CA125, the 24-25 kDa glycoprotein defined by MAb L6, or CEA, preferably a CEA.

6. A compound as claimed in any of claims 1 to 5, wherein the enzyme is a lactamase, preferably a Bacillus cereus II β-lactamase, pyroglutamate aminopeptidase, D-aminopeptidase, oxidase, peroxidase, phosphatase, hydroxynitrile lyase, protease, esterase, carboxypeptidase, preferably a carboxypeptidase G2 from Pseudomonas or glycosidase.

7. A compound as claimed in claim 6, wherein the enzyme is a β-glucuronidase, preferably an *E.coli*, *Kobayasia nipponica*, *Secale cereale* or human β-glucuronidase.

8. A compound as claimed in any of claims 1 to 7, wherein the antigen binding region is linked to the enzyme via a peptide linker.

9. A compound as claimed in any of claims 1 to 8, wherein the glycosylation takes place either by means of chemical methods or by a selection of suitable expression systems.

10. A compound as claimed in claim 1-9, which undergoes secretory expression in Saccharomyces cerevisiae or, more advantageously, in Hansenula polymorpha.

11. A compound as claimed in claim 1-9, which is expressed in E. coli arid is subsequently chemically glycosylated, preferably galactosylated and/or mannosylated.

12. A compound as claimed in claim 1-9, wherein the sFv-β-lactamase fusion protein, which has undergone periplasmic expression in E. coli, is chemically glycosylated, preferably galactosylated and/or mannosylated.

13. A compound as claimed in claim 1-9, wherein the sFv-β-lactamase fusion protein undergoes secretory expression in Saccharomyces cerevisiae or Hansenula polymorpha.

14. A nucleic acid coding for a compound as claimed in any of claims 1 to 8.

15. A nucleic acid as claimed in claim 14, coding for a humanized sFv fragment against CEA and a human β-glucuronidase.

16. A nucleic acid as claimed in claim 14 with the sequence shown in Tab. 1.

17. A vector containing a nucleic acid as claimed in any of claims 14 to 16.

18. A host cell containing a nucleic acid as claimed in any of claims 14 to 16 or a vector as claimed in claim 17.

19. A host cell as claimed in claim 18, which is a BHK, CHO, COS, HeLa, insect, tobacco plant, yeast or *E.coli* cell.

20. A transgenic mammal with the exception of a human, containing a DNA as claimed in any of claims 14 to 16 or a vector as claimed in claim 17.

21. A process for preparing a compound as claimed in claim 1 to 8, which comprises
a) introducing a nucleic acid as claimed in any of claims 14 to 16 or a vector as claimed in claim 17 into a host cell,
b) cultivating the host cell, and
c) isolating the compound.

22. A process for preparing a compound as claimed in claim 1 to 8, which comprises
a) cultivating a host cell as claimed in claim 18 or 19, and
b) isolating the compound.

23. The use of the compound as claimed in claim 1 to 13 for the preparation of a pharmaceutical or of a diagnostic aid.

24. The use of the compound as claimed in claim 1 to 13 for the preparation of a pharmaceutical for the treatment of cancer.

25. A pharmaceutical containing a compound as claimed in claim 1 to 13.

26. A diagnostic aid containing a compound as claimed in claim 1 to 13.

## Revendications

1. Composé contenant une région de liaison à l'antigène qui est liée à au moins une enzyme activant un précurseur de médicament, caractérisé en ce que la région de liaison à l'antigène consiste en une seule chaîne polypeptidique.

2. Composé selon la revendication 1, caractérisé en ce que le composé porte des glucides liés par covalence.

3. Composé selon les revendications 1 et 2, caractérisé en ce que la région de liaison à l'antigène contient un domaine variable d'une chaîne lourde d'anticorps et un domaine variable d'une chaîne légère d'anticorps (fragment sFv).

4. Composé selon la revendication 1 ou 2, caractérisé en ce que la région de liaison à l'antigène se lie à un antigène associé à une tumeur (TAA).

5. Composé selon la revendication 3, caractérisé en ce que le TAA est un antigène N-CAM, PEM, EGF-R, sialyl-Le^{a}, sialyl-Le^{x}, TFβ, GICA, GD₃, GD₂, TAG72, CA125, la glycoprotéine de 24-25 kDa définie par l'AcM L6, ou CEA, de préférence un antigène CEA.

6. Composé selon l'une des revendications 1 à 5, caractérisé en ce que l'enzyme est une lactamase, de préférence une β-lactamase II de *Bacillus cereus*, la pyroglutamate aminopeptidase, la D-aminopeptidase, une oxydase, peroxydase, phosphatase, hydroxynitrile lyase, protéase, estérase, carboxypeptidase, de préférence une carboxypeptidase G2 de *Pseudomonas*, ou une glycosidase.

7. Composé selon la revendication 6, caractérisé en ce que l'enzyme est une β-glucuronidase, de préférence une β-glucuronidase de *E. coli, Kobayasia nipponica*, *Secale cereale* ou la β-glucuronidase humaine.

8. Composé selon l'une des revendications 1 à 7, caractérisé en ce que la région de liaison à l'antigène est reliée à l'enzyme par un lien peptidique.

9. Composé selon l'une des revendications 1 à 8, caractérisé en ce que la glycosylation s'effectue soit par des méthodes chimiques, soit par le choix de systèmes d'expression appropriés.

10. Composé selon l'une des revendications 1 à 9, caractérisé en ce qu'il est exprimé par voie sécrétoire dans *Saccharomyces cerevisiae* ou plus avantageusement dans *Hansenula polymorpha.*

11. Composé selon l'une des revendications 1 à 9, caractérisé en ce qu'il est exprimé dans *E. coli* et est ensuite chimiquement glycosylé, de préférence galactosylé et/ou mannosylé.

12. Composé selon l'une des revendications 1 à 9, caractérisé en ce que la protéine de fusion sFv-β-lactamase, qui a été exprimée par voie périplasmique dans *E. coli*, est chimiquement glycosylée, de préférence galactosylée et/ou mannosylée.

13. Composé selon l'une des revendications 1 à 9, caractérisé en ce que la protéine de fusion sFv-β-lactamase est exprimée par voie sécrétoire dans *Saccharomyces cerevisiae* ou *Hansenula polymorpha*.

14. Acide nucléique, codant pour un composé selon l'une des revendications 1 à 8.

15. Acide nucléique selon la revendication 14, codant pour un fragment sFv humanisé dirigé contre CEA et pour une β-glucuronidase humaine.

16. Acide nucléique selon la revendication 14, ayant la séquence selon le tableau 1.

17. Vecteur, contenant un acide nucléique selon l'une des revendications 14 à 16.

18. Cellule hôte, contenant un acide nucléique selon l'une des revendications 14 à 16 ou un vecteur selon la revendication 17.

19. Cellule hôte selon la revendication 18, caractérisée en ce qu'elle est une cellule BHK, CHO, COS, HeLa, d'insecte, de plant de tabac, de levure ou de *E. coli*.

20. Mammifère transgénique, à l'exception de l'homme, contenant un ADN selon l'une des revendications 14 à 16 ou un vecteur selon la revendication 17.

21. Procédé pour la production d'un composé selon l'une des revendications 1 à 8, caractérisé en ce que
a) un acide nucléique selon l'une des revendications 14 à 16 ou un vecteur selon la revendication 17 est introduit dans une cellule hôte,
b) la cellule hôte est cultivée et
c) le composé est isolé.

22. Procédé pour la production d'un composé selon l'une des revendications 1 à 8, caractérisé en ce que
a) une cellule hôte selon la revendication 18 ou 19 est cultivée et
b) le composé est isolé.

23. Utilisation du composé selon l'une des revendications 1 à 13, pour la fabrication d'un médicament ou d'un agent de diagnostic.

24. Utilisation du composé selon l'une des revendications 1 à 13, pour la fabrication d'un médicament destiné au traitement du cancer.

25. Médicament, contenant un composé selon l'une des revendications 1 à 13.

26. Agent de diagnostic, contenant un composé selon l'une des revendications 1 à 13.
